(19) Europäisches Patentamt
European Patent Office
Office européen des brevets

(11) **EP 1 955 045 B1**

(12) **EUROPÄISCHE PATENTSCHRIFT**

(45) Veröffentlichungstag und Bekanntmachung des Hinweises auf die Patenterteilung:
**25.08.2021 Patentblatt 2021/34**

(21) Anmeldenummer: **06830015.1**

(22) Anmeldetag: **16.11.2006**

(51) Int Cl.:
*G01N 15/14* (2006.01)    *G01N 15/00* (2006.01)
*G01N 33/00* (2006.01)

(86) Internationale Anmeldenummer:
**PCT/EP2006/068541**

(87) Internationale Veröffentlichungsnummer:
**WO 2007/060127 (31.05.2007 Gazette 2007/22)**

(54) **VORRICHTUNGEN UND VERFAHREN FÜR DIE AUTOMATISCHE BESTIMMUNG DER INDIVIDUELLEN DREIDIMENSIONALEN PARTIKELFORM**

DEVICES AND METHODS FOR THE AUTOMATIC DETERMINATION OF THE INDIVIDUAL THREE-DIMENSIONAL SHAPE OF PARTICLES

PROCÉDÉS ET DISPOSITIFS POUR LA DÉTERMINATION AUTOMATISÉE DE LA FORME TRIDIMENSIONNELLE INDIVIDUELLE DE PARTICULES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **23.11.2005 DE 102005055825**

(43) Veröffentlichungstag der Anmeldung:
**13.08.2008 Patentblatt 2008/33**

(73) Patentinhaber: **BASF SE**
**67056 Ludwigshafen am Rhein (DE)**

(72) Erfinder:
• SCHAEFER, Michael
  67122 Altrip (DE)
• ETTMUELLER, Juergen
  67454 Hassloch (DE)
• ZIEGLER, Stefan
  76857 Eusserthal (DE)
• REINDEL, Klaus
  67454 Hassloch (DE)

(56) Entgegenhaltungen:
EP-A- 1 662 247        EP-A2- 1 464 949
WO-A-2005/062022      WO-A2-02/11065
DE-A1- 2 445 148      JP-A- H11 258 144
US-A- 4 975 863        US-A- 4 999 513
US-A- 5 644 388        US-A- 6 049 381
US-A1- 2003 053 065

• KACHEL V ET AL: "UNIFORM LATERAL ORIENTATION, CAUSED BY FLOW FORCES, OF FLAT PARTICLES IN FLOW-THROUGH SYSTEMS" JOURNAL OF HISTOCHEMISTRY AND CYTOCHEMISTRY, HISTOCHEMICAL SOCIETY, NEW YORK, NY, US, Bd. 25, Nr. 7, 1977, Seiten 774-780, XP002915897 ISSN: 0022-1554 in der Anmeldung erwähnt

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft ein Verfahren zur automatisierten Bestimmung der individuellen dreidimensionalen Form von Partikeln, umfassend die folgenden Schritte: a) Dosierung, Ausrichtung und automatisierte Förderung der Partikeln; b) Beobachtung der ausgerichteten Partikeln und Bilderfassung und c) Auswertung der Bilder. Des Weiteren betrifft die vorliegende Erfindung eine Vorrichtung zur automatisierten Bestimmung der individuellen dreidimensionalen Form von Partikeln umfassend: a) Mittel zum Dosieren, Ausrichten und automatisierten Fördern der Partikeln; b) mindestens zwei Kameras zur Beobachtung der ausgerichteten Partikeln und c) Mittel zur Auswertung der Bilder; sowie die Verwendung der erfindungsgemäßen Vorrichtung zur automatisierten Bestimmung der individuellen dreidimensionalen Form von Partikeln.

Unter Form ist im Sinne der vorliegenden Anmeldung die räumliche Form, d.h. einerseits die Form von Partikeln im mathematischen Sinne zu verstehen, d.h. die Beschreibung der Partikelabmessungen z.B. durch geometrische Abmessungen (Beispiele: Länge, Durchmesser) und Körper (Beispiele: Kugel, Ellipsoid, Zylinder, Quader) und deren Maße, und andererseits die "freie" Beschreibung (der Oberfläche oder des Volumens beliebiger Körper) durch finite Flächenelemente wie Dreiecke, oder durch diskrete Volumenelemente wie kubische Voxel oder andere aus der Kristallographie bekannte Raumgitterelemente oder durch Kugeln. Diese beiden Formbeschreibungen im engeren Sinne, deren Wirklichkeitsnähe nur durch die Auflösung begrenzt wird, ist nicht zu verwechseln mit den in der Literatur oft erwähnten Formparametern, die nur eine unvollständige und in der Regel nicht physikalisch interpretierbare Aussage über die Form machen. Aus der vollständigen Formbeschreibung (Gestalt) ergeben sich auch das Partikelvolumen und die Oberfläche und daraus abgeleitet die Scheindichte, die Porosität, die spezifische Oberfläche sowie alle die Größe und Form der Partikeln beschreibenden Parameter (wie diese Parameter an einer bekannten Form zu bestimmen sind, ist dem Fachmann bekannt). Weiterhin kann dieses Verfahren auch erweitert werden, und zusätzlich zur gleichzeitigen Bestimmung von Oberflächeneigenschaften wie Oberflächenrauhigkeit und Farbe (sowie Farbhomogenität) von Partikeln eingesetzt werden, sowie zur Detektion von anhaftenden weiteren Partikeln, Oberflächendefekten und Einschlüssen, und zur Beurteilung von Beschichtungsqualität und -homogenität.

Die Partikelform, insbesondere bei beliebig geformten, nicht-kugelförmigen Partikeln, ist ein wesentlicher Parameter zur Beurteilung der Produktqualität und des Verhaltens solcher Partikeln im Prozess. Insbesondere bei Schüttungen mit beliebig geformten Partikeln ist die Partikelform ein wesentlicher Parameter. Solche Schüttungen spielen in der mechanischen und chemischen Verfahrenstechnik eine wesentliche Rolle. So wird das Verhalten der Schüttungen im Wesentlichen durch die Packungsdichte eines Systems und dessen Struktur beeinflusst. Die Packungsdichte und Struktur einer Schüttung werden wiederum wesentlich durch die Partikelform beeinflusst. Somit hat die Partikelform beliebig geformter Partikel einen wesentlichen Einfluss, z. B. auf die erzielbare Schüttdichte, das Staubabscheideverhalten, die Wärmeleitung oder den Druckwiderstand von durchströmten Schüttungen, das Festigkeitsverhalten von Agglomeraten sowie das Entfeuchtungsverhalten von Filterkuchen. Die grundsätzlichen Zusammenhänge zwischen Form und anderen Partikeleigenschaften einerseits und Produkteigenschaften und Prozessverhalten sind dem Fachmann unter den Begriffen Eigenschaftsfunktion und Prozessfunktion bekannt (Schubert, Heinrich (Herausgeber); Handbuch der Mechanischen Verfahrenstechnik, Band 1, Kapitel 2). Im konkreten Einzelfall müssen Sie messtechnisch bestimmt oder durch Modelle berechnet werden.

Eine präzise, vollständige und unverfälschte Kenntnis der individuellen Partikelform von beliebig geformten Partikeln (d.h. des Volumens, der Form im engeren Sinne, der Größe und der Scheindichte der Partikel) sind somit Voraussetzungen für die Optimierung von Produkteigenschaften und Verfahren, sowie für Simulationsrechnungen auf Partikelebene.

Es sind bereits Verfahren kommerziell verfügbar, die Partikeln abbilden, und eine Formcharakterisierung auf Basis einer Projektionsfläche in zufälliger Orientierung oder auch in stabiler Lage ermöglichen. Diese mit zweidimensionaler Formbeschreibung (2D) bezeichneten Methoden liefern eine sehr begrenzte Forminformation, die die oben genannten Anforderungen bei Weitem nicht erfüllen kann.

Beispielsweise ist eine Formcharakterisierung von Partikeln in zufälliger Orientierung während des freien Falls der Partikeln bekannt. Stellvertretend für eine Reihe von kommerziell verfügbaren Geräten mit freiem Fall der Partikeln sei hier das Gerät CamSizer® von Retsch genannt. Es sei darauf hingewiesen, dass die in diesem Gerät verwendeten zwei Kameras nur der Erweiterung des Messbereiches dienen und nicht zur Verbesserung der Formerfassung.

Stellvertretend für Geräte zur Analyse von Partikeln, die in stabiler Lage auf einem Substrat liegen, sei das Gerät Pharmavision® von Malvern genannt.

Schließlich sind noch Verfahren bekannt, die Partikeln in einer Strömungszelle ausrichten, und aus einer Richtung abbilden.

DE-A 24 45 148 betrifft eine Vorrichtung zum Ausrichten von Teilchen in einer Suspension, insbesondere eine Vorrichtung zum Ausrichten von im Allgemeinen flachen Teilchen in einer Stellung, die zu ihrem Abtasten beim Passieren einer Kontrollvorrichtung in einem Schlitzverschluss-Lichtmessinstrument geeignet ist.

Kachel V et al., Journal of Histochemistry and Cytochemistry, 25(7), 774 bis 780, 1977 betrifft eine Vorrichtung zur einheitlichen lateralen Orientierung von flachen Partikeln in Durchflusssystemen, wobei die einheitliche Orientierung durch Fließkräfte erreicht wird.

Kommerziell ist z.B. ein Gerät von Sysmex bekannt.

Alle diese Verfahren und Geräte haben die Erfassung aus einer Richtung mit einem Flächensensor gemeinsam, was hier zusammenfassend 2D genannt und nicht weiter betrachtet werden soll, da die Erfindung ein 3D Verfahren und ein Gerät mit mindestens 2 Beobachtungsrichtungen betrifft.

Grundsätzlich sind verschiedene Verfahren zur räumlichen (3D) Abbildung von Partikeln denkbar.

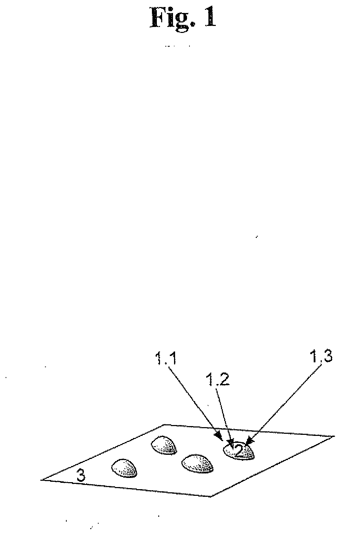

In Figur 1 sind Untersuchungsmöglichkeiten für auf einem flächigen Präparat aufgebrachte Partikeln dargestellt.

Gemäß Figur 1 kann eine 3D-Analyse einer auf einem flächigen Präparat abgelegten Partikel erfolgen, indem aus mehreren Winkeln beobachtet wird.

Im Stand der Technik werden Ausführungen und Methoden gemäß Figur 1 beschrieben, worin einzelne Partikeln oder kleinere Gruppen, die auf einem Substrat liegen, aus drei orthogonalen Richtungen abgebildet werden. Aus den drei erhaltenen Bildern wird die dreidimensionale Form rekonstruiert (z.B. R. Weichert und D. Huller: Volumenbestimmung und Formerkennung unregelmäßig geformter Partikeln mittels dreidimensionaler Bildanalyse; Nürnberg, 2. Europ. Symposium Partikelmesstechnik (1979), S. 266-272). Dieses Verfahren liefert bei rundlichen Partikeln eine gute Forminformation, aber bei beliebigen, nicht-rundlichen Formen wird die Mehrzahl der Partikeln unter ungünstigen Winkeln erfasst, so dass die tatsächliche Form verborgen bleibt. Des Weiteren ist das Verfahren auf Grund der aufwändigen Präparation und Auswertung nur für sehr kleine Partikelzahlen wirtschaftlich tragbar.

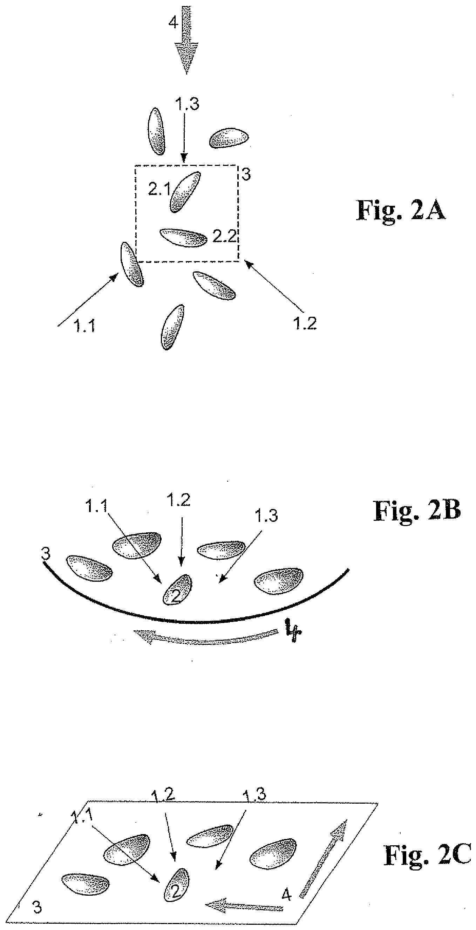

In Figur 2 sind Möglichkeiten der on-line Abbildung (bzw. automatisierten Abbildung) von Partikeln dargestellt, die auf dem in Figur 1 dargestellten Verfahren 3D-Bildanalyse beruhen (Beobachtung unter verschiedenen Winkeln).

Gemäß Figur 2A erfolgt eine 3D-Bildanalyse von im freien Fall befindlichen Partikeln Gemäß Figur 2B erfolgt eine 3D-Bildanalyse von auf einem Drehteller einachsig ausgerichteten Partikeln.

Gemäß Figur 2C erfolgt eine 3D-Bildanalyse von auf einem X/Y-Scantisch einachsig ausgerichteten Partikeln.

WO 02/11065 A2 betrifft u. a. ein System zur Aufnahme von zahlreichen Bildern eines Objekts, das eine Vorrichtung zur Zuführung des Objekts in die Aufnahmezone umfasst, wobei das Objekt einen vorbestimmten Punkt in der Aufnahmezone durchläuft, eine erste und eine zweite Aufnahmevorrichtung, wobei die zweite Aufnahmevorrichtung im Wesentlichen im 90°-Winkel zur ersten Aufnahmevorrichtung angeordnet ist, wobei das System des Weiteren eine Förderrinne in Kombination mit einem Förderband aufweisen kann. Mithilfe des Systems in WO 02/11065 A2 kann ein dreidimensionales Bild eines Partikels ermittelt werden. Aus der Offenbarung in WO 02/11065 A2 geht hervor, dass sich die zu messenden Partikel im freien Fall durch die Aufnahmezone bewegen.

EP 1 464 949 A2 betrifft eine Einrichtung zur Analyse von Partikeln, die eine Dosiereinrichtung aufweist, die geeignet ist, ein Partikel in eine Beobachtungszone zu führen (to drop), einen Reflektor, der geeignet ist, ein reflektiertes Bild des Partikels in der Beobachtungszone bereitzustellen, wobei das Bild des Partikels mindestens eine direkte Blickrichtung und mindestens eine reflektierte Blickrichtung des Partikels umfasst. In EP 1 464 949 A2 wird ein fallendes Teilchen beobachtet.

US 5,644,388 A betrifft ein Verfahren zur Bestimmung des Bilds eines Partikels in einer Durchflusszelle, wobei die Bestimmung durch Messung der Lichtstreuung von mit z. B. Fluoreszenzmarkern markierten Partikeln erfolgt. Bei dem Partikelstrom in der Fließzelle handelt es sich bevorzugt um eine einzige Linie, die von einem Hüllstrom umhüllt ist.

US 6,049,381 A betrifft eine Vorrichtung zur Beobachtung von suspendierten Partikeln in einem Fluid in Echtzeit, umfassend eine Laser-Lichtquelle, eine Vorrichtung zur parallelen Ausrichtung eines optischen Strahls der Lichtquelle, eine Fließzelle, eine Vorrichtung, um ein optisches Bild zu bilden, Vorrichtungen zur Klassifizierung der Formen von Partikeln, wobei die Vorrichtung zur Klassifizierung der Formen von Partikeln einen bestimmten Aufbau aufweist.

Die Veröffentlichung von Kachel et al., "UNIFORM LATERAL ORIENTATION, CAUSED BY FLOW FORCES, OF FLAT PARTICLES IN FLOW-THROUGH SYSTEMS" (THE JOURNAL OF HISTOCHEMISTRY AND CYTOCHEMISTRY, 1977, Band 25, Nr. 7, Seiten 774-780) offenbart eine Ausrichtung der Partikeln in Längsachse in einer Strömungszelle zur zweiachsigen Ausrichtung von Partikeln.

US 4,999,513 A betrifft eine Vorrichtung zur Analyse von Partikeln umfassend eine Vorrichtung zur Vereinzelung der Partikeln, die untersucht werden sollen, wobei die Partikel, die untersucht werden sollen, mit Licht aus zwei Richtungen bestrahlt werden, wobei die erste und die zweite Richtung unterschiedlich sind, sowie ein erstes und ein zweites Photometer, das von den Partikeln abgestrahltes Licht aufnimmt. Die Partikeln durchlaufen eine Fließzelle, wobei das Prinzip eines laminaren Hüllstroms angewandt wird.

EP 1 662 247 A1, das vor dem Internationalen Anmeldedatum, aber nach dem beanspruchten Prioritätsdatum der vorliegenden Anmeldung veröffentlicht wurde, betrifft ein Verfahren zur Bestimmung der Anzahl und/oder der Partikelform und/oder der Größe von landwirtschaftlichen Gutpartikeln wie Saatkörner, Düngerkörner, Spritzbrühetropfen, Getreidekörner, Getreidestroh, etc. durch eine zeilenweise optoelektronische Abtastung eines Partikelstroms, bei dem der Partikelstrom in einem transparenten Medium durch Schwerkraft oder zusätzliche Kräfte an einer optoelektischen Messstrecke senkrecht zu parallelen Lichtstrahlen vorbeibewegt wird, und bei dem die Signale der abgedeckten und nicht abgedeckten Elemente der jeweiligen CCD-Zeile in einer elektronischen Auswerte-Einheit ausgewertet und in einem Speichermedium gespeichert werden, und zwar in zeitlicher Reihenfolge hintereinander, so dass ein zeitlich ablaufender Videofilm über die die Messstrecke passierenden Gutpartikel entsteht.

WO 2005/062022 A betrifft eine Strömungszelle zur zweiachsigen Ausrichtung von Partikeln, die in einer Dispersion vorliegen. Ein Verfahren zur automatisierten Bestimmung der individuellen dreidimensionalen Form von Partikeln sowie eine entsprechende Vorrichtung und deren Verwendung sind in WO 2005/062022 A nicht offenbart.

JPH11258144 A betrifft eine Fließzelle, die eine Partikel-enthaltende flüssige Probe enthält, die von einem Hüllstrom umschlossen ist. In JPH11258144 A ist erwähnt, dass eine Messung der Probe gemäß JPH11258144 A aus zwei Richtungen deswegen möglich ist, da die Distanz zwischen den Messpositionen B1 und B2 sehr kurz ist, so dass keine Rotation der Probe zu erwarten ist. Demnach lehrt die JPH11258144 A, dass eine Messung von Partikeln einer Probe in Form von Dispersionen in zwei Richtungen dann möglich ist, wenn die in unterschiedlichen Richtungen angebrachten Messeinheiten in Fließrichtung nahe beieinander sind, um eine Rotation der Probenpartikeln auszuschließen.

In dem Dokument US 4,975,863 A ist ein System zur Untersuchung von Partikeln beschrieben, dass die gewünschten Eigenschaften eines jeden Partikel aus einer Gruppe von Partikeln automatisch ohne menschliche Überprüfung aufnimmt. Dabei werden die Partikeln für die Prüfung orientiert. Die Orientierung erfolgt bevorzugt durch eine automatische Ausrichtung mit Hilfe eines sogenannten Singulators 9. Der Singulator umfasst einen hohlen Körper 10 mit einer inneren Öffnung 11. Die innere Öffnung 11 kann mit einer Vakuumquelle durch eine Vakuumverbindung 12 verbunden werden. Das Oberteil des hohlen Körpers wird durch eine Oberfläche 7 gebildet, die mindestens ein Loch 13 aufweist, das in Kontakt mit der inneren Öffnung 11 steht. Wenn nun ein Vakuum an der inneren Öffnung 11 angelegt wird und Partikeln 6 auf die Oberfläche 7 gegeben werden, werden die Partikeln an der Stelle über den Löchern 13 gehalten. Die Löcher 13 müssen kleiner sein als die zu untersuchenden Partikeln 6. Die Löcher 13 sind so angeordnet, dass eine gleichmäßige Verteilung der Partikeln 6 über die Oberfläche 7 erfolgt und sich die einzelnen Partikeln nicht berühren. Eine zusätzliche Ausrichtung wird durch sogenannte "grooves" 14 (Rillen) erreicht, die die Partikeln 6 in der gleichen Richtung orientieren, um ein akkurates Videoscanning zu ermöglichen.

In US 2003/0053065 A1 ist eine Vorrichtung zur Bestrahlung von kleinen Partikeln zur Analyse der Qualität der Partikeln offenbart. Diese Vorrichtung umfasst einen Probenzufuhrträger (sample-feeding carrier), der einen Probenhalter umfasst und so angepasst ist, dass er Partikelproben aufnehmen kann, die jeweils mindestens ein Partikel enthalten, wobei die Partikeln in den Partikelhaltern zu dem Ort der Bestrahlung transportiert werden. Jeder Probenhalter hat bevorzugt eine Form, die der des Partikels entspricht, um die Orientierung des Partikels in dem Probenhalter zu kontrollieren.

Ein etabliertes Verfahren zur Erfassung der Form von Objekten ist natürlich auch die Röntgentomographie. Diese wird sowohl in der Medizin, als auch in der Fertigungsindustrie eingesetzt. Die Röntgentomographie ist zwar die vollständigste denkbare Forminformation, aber der apparative und zeitliche Aufwand (und damit die Kosten) sind so groß, dass dieses Verfahren in der Partikelmesstechnik nicht routinemäßig anwendbar ist.

Für die Beschreibung von Partikelkollektiven, z.B. in Form von Schüttungen, müssen für jede Probe große Partikelzahlen erfasst werden, bei sehr ähnlichen, einfach geformten Partikeln mehrere hundert bis mehrere tausend, bei in Größe und Form stark variierenden oder komplex geformten Partikeln mehrere tausend bis zehntausend. Von jeder Partikel muss die vollständige räumliche Form, z. B. in digitalisierter Darstellung, mit einer hinreichenden Auflösung erfasst werden.

Aufgabe der vorliegenden Anmeldung ist daher die Bereitstellung eines Verfahrens zur automatisierten Bestimmung der individuellen dreidimensionalen Form von Partikeln, wobei in kurzer Zeit die individuelle dreidimensionale Form auch von komplex geformten Partikeln ermittelt werden kann, so dass eine Anwendung dieses Verfahrens in der Partikelmesstechnik möglich ist.

Diese Aufgabe wird gelöst durch ein Verfahren zur automatisierten Bestimmung der individuellen dreidimensionalen Form von rieselfähigen Partikeln von Proben in Pulverform, wobei die folgenden Schritte nacheinander durchgeführt werden:

a) Vereinzelnde Dosierung der Partikeln, Ausrichtung der Partikeln in Längsachse und automatisierte Förderung der Partikeln entlang einer Linie;

b) Beobachtung der ausgerichteten Partikeln aus mindestens zwei Beobachtungsrichtungen und Bilderfassung; wobei die Beobachtung mit Hilfe von mindestens zwei Kameras erfolgt;

c) Auswertung der Bilder,

dadurch gekennzeichnet, dass die Ausrichtung von Partikeln von Proben in Pulverform gegen zwei plane Anlageflächen, die eine Förderrinne bilden, mit Hilfe der Schwerkraft oder von Zentrifugalkräften erfolgt, und eine Ausrichtung der Partikeln in allen Achsen erfolgt.

Des Weiteren wird diese Aufgabe gelöst durch ein Verfahren zur automatisierten Bestimmung der individuellen dreidimensionalen Form von Partikeln von Proben in Form von Dispersionen, wobei die folgenden Schritte nacheinander durchgeführt werden:

a) Vereinzelnde Dosierung der Partikeln, Ausrichtung der Partikeln in Längsachse und automatisierte Förderung der Partikeln entlang einer Linie;

b) Beobachtung der ausgerichteten Partikeln aus mindestens zwei Beobachtungsrichtungen und Bilderfassung; wobei die Beobachtung mit Hilfe von mindestens zwei Kameras erfolgt;

c) Auswertung der Bilder,

dadurch gekennzeichnet, dass die Ausrichtung und automatisierte Förderung von Partikeln von Proben in Form von Dispersionen in einer Strömungszelle zur zweiachsigen Ausrichtung von Partikeln, umfassend eine Zulaufzone für die Probe enthaltend auszurichtende Partikel und einen Auslass für die Probe enthaltend in zwei Achsen ausgerichtete Partikel, wobei ein Fluidelement der Probe mit den Maßen a, b, c in einer Dehnungszone zu einem Fluidelement mit den Maßen $a \times n$, $b/(n \times m)$, $c \times m$ umgeformt wird, wobei a die Breite, b die Höhe und c die Länge des Fluidelements bedeuten, und n und m von der Geometrie der Strömungszelle abhängige Konstanten (Dehnungsgrad) sind, die positive Zahlen > 1 bedeuten, erfolgt.

Des Weiteren betrifft die vorliegende Erfindung eine Vorrichtung zur automatisierten Bestimmung der individuellen dreidimensionalen Form (s.o.) von rieselfähigen Partikeln von Proben in Pulverform umfassend:

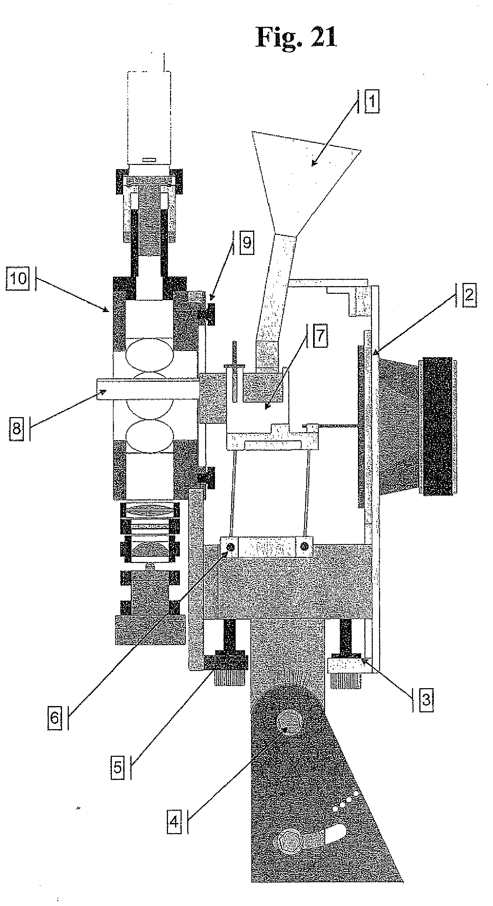

a) Mittel zum vereinzelnden Dosieren der Partikeln (1, Fig. 21), Mittel zum Ausrichten der Partikeln in Längsachse und Mittel zum automatisierten Fördern der Partikeln entlang einer Linie (8, Fig. 21);

b) mindestens zwei Kameras zur Beobachtung der ausgerichteten Partikeln aus mindestens zwei Beobachtungsrichtungen (10, Fig. 21);

c) Mittel zur Auswertung der Bilder,

dadurch gekennzeichnet, dass die Mittel zum Ausrichten der Partikeln in Längsachse dazu eingerichtet sind, dass die Ausrichtung von Partikeln von Proben in Pulverform gegen zwei plane Anlageflächen, die eine Förderrinne bilden, mit Hilfe der Schwerkraft oder von Zentrifugalkräften, erfolgt.

Weiterhin betrifft die vorliegende Erfindung eine Vorrichtung zur automatisierten Bestimmung der individuellen dreidimensionalen Form von Partikeln von Proben in Form von Dispersionen umfassend:

a) Mittel zum vereinzelnden Dosieren der Partikeln (1, Fig. 21), Mittel zum Ausrichten der Partikeln in Längsachse und Mittel zum automatisierten Fördern der Partikeln entlang einer vorgegebenen Linie (8, Fig. 21);

b) mindestens zwei Kameras zur Beobachtung der ausgerichteten Partikeln; aus mindestens zwei Beobachtungsrichtungen (10, Fig. 21)

c) Mittel zur Auswertung der Bilder,

dadurch gekennzeichnet, dass die Mittel zum Ausrichten der Partikeln in Längsachse dazu eingerichtet sind, dass die Ausrichtung und automatisierte Förderung von Partikeln von Proben in Form von Dispersionen in einer Strömungszelle zur zweiachsigen Ausrichtung von Partikeln, umfassend eine Zulaufzone für die Probe enthaltend auszurichtende Partikel und einen Auslass für die Probe enthaltend in zwei Achsen ausgerichtete Partikel, wobei ein Fluidelement der Probe mit den Maßen a, b, c in einer Dehnungszone zu einem Fluidelement mit den Maßen $a \times n$, $b/(n \times m)$, $c \times m$ umgeformt wird, wobei a die Breite, b die Höhe und c die Länge des Fluidelements bedeuten, und n und m von der Geometrie der Strömungszelle abhängige Konstanten (Dehnungsgrad) sind, die positive Zahlen > 1 bedeuten, erfolgt.

**Zeichnung**

Anhand der Zeichnung werden der Stand der Technik und die Erfindung näher erläutert: Es zeigen:

Figur 1: Untersuchungsmöglichkeiten für auf einem flächigen Präparat abgelegte Partikeln, wobei aus mehreren Winkeln beobachtet wird;

| Figur 2: | Möglichkeiten der on-line Abbildung (bzw. der automatisierten Abbildung) von Partikeln; Figur 2A: 3D-Bildanalyse von im freien Fall befindlichen Partikeln; Figur 2B: 3D-Bildanalyse von auf einem Drehteller einachsig ausgerichteten Partikeln; Figur 2C: 3D-Bildanalyse von auf einem X/X-Scantisch einachsig ausgerichteten Partikeln; |
|---|---|
| Figur 3: | Ausführungsbeispiel einer erfindungsgemäßen Vorrichtung mit trockener Förderung und 4 Kameras (Vorderansicht); |
| Figur 4: | Ausführungsform einer erfindungsgemäßen Vorrichtung zur Dosierung von Partikeln pulverförmiger Proben sowie verschiedene Ansichten eines Trichters zur Produktführung; |
| Figuren 5 bis 18 und 23: | bevorzugte Ausführungsformen der Erfindung bezüglich der Förderung der Partikeln und der Zahl, Art und Anordnung der eingesetzten Kameras; |
| Figur 19: | Figur zur Erläuterung der Ermittlung von Schnittpunkten zwischen den Beobachtungsstrahlen bei einer Ausführungsform mit Beobachtung aus 4 Beobachtungsrichtungen zur Ermittlung des Volumens einer individuellen Partikel; |
| Figur 20: | Figur zur Erläuterung der Ermittlung des maximalen, minimalen und tatsächlichen Querschnitts einer an einer definierten Position einer individuellen Partikel befindlichen Scheibe durch Beschreibung der Querschnitte durch 8-ecke, bei der in Figur 19 dargestellten Ausführungsform mit Beobachtung aus 4 Beobachtungsrichtungen zur Ermittlung des Volumens einer individuellen Partikel; |
| Figur 21: | Ausführungsform der erfindungsgemäßen Vorrichtung (Vorderansicht); |
| Figur 22: | Ausführungsform der erfindungsgemäßen Vorrichtung (Optik-Block, Aufsicht); |
| Figur 24: | In den Varianten 1 bis 5 (Figuren 5 bis 18 und 23) eingesetzte Materialien. |

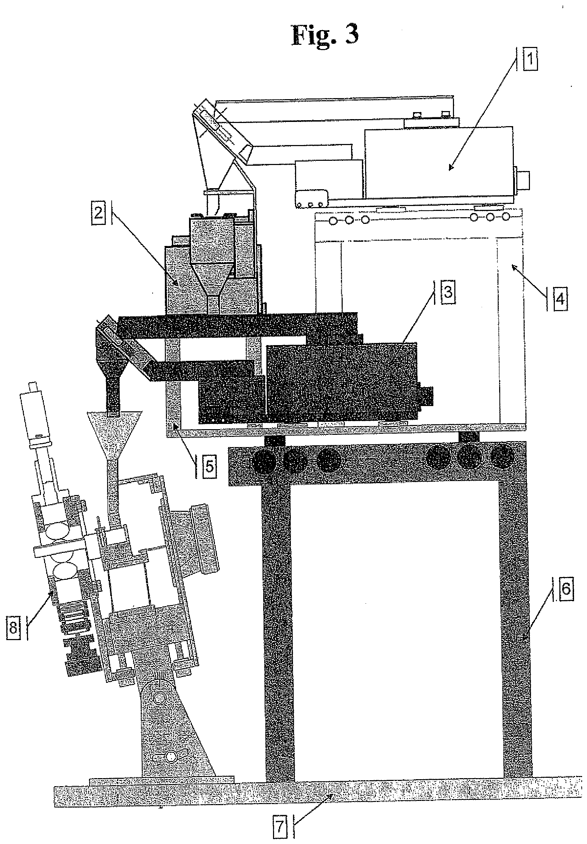

Eine Übersicht über eine erfindungsgemäß realisierte Vorrichtung mit trockener Förderung und 4 Kameras ist in Figur 3 dargestellt.

Mit Hilfe des erfindungsgemäßen Verfahrens bzw. der erfindungsgemäßen Vorrichtung ist es möglich, in kurzer Zeit die dreidimensionale Form von Partikeln für große Partikelzahlen zu erfassen, wobei eine Darstellung mit einer hinreichenden Auflösung und handhabbaren Datenmengen erfolgt. Weiterhin können mit Hilfe des erfindungsgemäßen Verfahrens bzw. der erfindungsgemäßen Vorrichtung neben der individuellen dreidimensionalen Form der Partikeln in speziellen Ausführungsformen spezielle Produkteigenschaften (z.B. Größenbereiche, Formklasse, optische Materialeigenschaften und Rieselfähigkeit) ermittelt und spezielle Fragestellungen (z.B. Form, Größe, Gesamtvolumen, Farbe) beantwortet werden.

a) Dosierung, Ausrichtung und automatisierte Förderung der Partikeln

Die Vorgänge der Dosierung, Ausrichtung und automatisierten Förderung können nacheinander erfolgen. Es ist ebenfalls möglich, dass zwei oder mehr der Vorgänge gleichzeitig erfolgen. In einer bevorzugten Ausführungsform erfolgen zum Beispiel die Ausrichtung und die automatisierte Förderung gleichzeitig, das heißt, in einem Verfahrensschritt und unter Einsatz eines Mittels zur Ausrichtung und automatisierten Förderung.

Wird im Folgenden der Begriff "Probe" verwendet, so bedeutet dieser Begriff im Sinne der vorliegenden Anmeldung eine Mehrzahl von zu untersuchenden Partikeln. Die Probe kann in verschiedenen Formen vorliegen, wie nachstehend ausgeführt ist.

Die zu messende Probe kann in verschiedenen Formen vorliegen, z.B. in Form eines Pulvers oder in Form einer Dispersion, wobei die zu vermessenden Partikeln der Probe in einer Flüssigkeit dispergiert sind. Handelt es sich bei der Probe um rieselfähige Partikeln, also um Partikeln, die vereinzelbar sind und nicht aneinander haften, erfolgt die erfindungsgemäße Bestimmung der individuellen dreidimensionalen Form der Partikeln im Allgemeinen anhand einer Probe in Form eines Pulvers. Viele Produkte sind noch bei Partikelgrößen von etwa 20 $\mu$m rieselfähig, so dass diese Proben in Pulverform untersucht werden können. Die genaue Grenze, ob ein Pulver rieselfähig ist oder nicht, hängt von der Oberflächenbeschaffenheit, der Form und der Dichte der Partikeln ab. Die Zusammenhänge sind dem Fachmann bekannt. Sehr leichte oder gar klebrige Partikeln, die nicht gut rieselfähig sind, können z.B. in Form ihrer Dispersionen untersucht werden.

Die Dosierung, Ausrichtung und automatisierte Förderung der Partikeln und entsprechende Mittel zur Dosierung, Ausrichtung und automatisierten Förderung der Partikeln sind abhängig davon, in welcher Form (z.B. Pulverform oder Dispersion) die Probe vorliegt. Geeignete bevorzugte Ausführungsformen und Mittel zur Dosierung, Ausrichtung und automatisierten Förderung von Partikeln in Proben, die in verschiedenen Formen vorliegen, sind nachstehend genannt.

aa) *Dosierung*

Bei der Dosierung der Partikeln erfolgt ein Aufbringen der Partikeln auf eine Förderstrecke zur automatisierten Förderung der Partikeln. Der Mechanismus für Dosierung und Förderung (siehe unten) kann der gleiche sein.

Erfindungsgemäß erfolgt eine vereinzelnde Dosierung. Dabei bedeutet der Begriff "vereinzelnd", dass die einzelnen Partikeln einer zu vermessenden Probe, die eine Mehrzahl von Partikeln enthält, separiert voneinander vorliegen und bevorzugt einzeln auf die Förderstrecke aufgegeben werden, so dass eine Beobachtung jeder individuellen Partikel der Probe in Schritt b) des erfindungsgemäßen Verfahrens ermöglicht wird. Die Durchführung der vereinzelnden Dosierung sowie geeignete Mittel zur vereinzelnden Dosierung sind unter anderem davon abhängig, in welcher Form die Probe vorliegt.

i) Vorliegen der Probe in Pulverform (trockene Förderung)

Bei einer trockenen Förderung sind verschiedne Ausführungsformen einer vereinzelnden Dosierung möglich, wobei die folgenden Ausführungsformen bevorzugt sind:

ia) "voreingestellte" Zeitabstände

Die vereinzelnde Dosierung kann in einer Ausführungsform in der Art erfolgen, dass jeweils definierte Zeitabstände eingestellt werden, wobei zu bestimmten voreingestellten Zeitpunkten jeweils eine Partikel zudosiert wird. Beispielsweise kann eine Voreinstellung in der Form vorgenommen werden, dass jeweils eine Partikel pro Sekunde zudosiert wird. Diese Zeitvorgabe ist lediglich beispielhaft. Grundsätzlich können - in Abhängigkeit von der Probe und anderen Parametern - beliebige Zeitabstände voreingestellt werden.

ib) "zufällige" Zeitabstände

In einer weiteren Ausführungsform kann die vereinzelnde Dosierung in der Art vorgenommen werden, dass die Partikeln in zufälligen Zeitabständen dosiert werden. Dabei sind die Zeitabstände unter anderem von der Art der Dosierung und der Art der Partikeln in der zu messenden Probe abhängig. Die Zudosierung der einzelnen Partikeln erfolgt bei dieser Ausführungsform nicht in definierten Zeitabständen, sondern die Zeitabstände der Dosierung der einzelnen Partikeln können jeweils gleich oder unterschiedlich sein.

ic) "particle on demand"

In einer weiteren Ausführungsform können die Partikeln "auf Abruf" dosiert werden. Dabei ist es möglich, jeweils eine neue zu messende Partikel dann zu dosieren, wenn ein Vorgang, zum Beispiel die Speicherung der Daten der vorausgegangenen Partikel oder die Verarbeitung der Daten der vorausgegangenen Partikel ("online Verarbeitung") abgeschlossen ist.

Bevorzugte Ausführungsformen der vereinzelnden Dosierung bei der trockenen Förderung sind die Ausführungsformen ia) und ic). Besonders bevorzugt ist die Ausführungsform ic).

ii) Vorliegen der Probe in Form einer Dispersion (nasse Förderung)

Bei einer nassen Förderung erfolgt die vereinzelnde Dosierung der Partikeln im Allgemeinen in "zufälligen" Zeitabständen (iib) in Abhängigkeit von der Strömung der die Partikeln enthaltenden Dispersion durch die eingesetzte Strömungszelle.

Anstelle der Führung einer Dispersion enthaltend die zu messenden Partikeln durch die Messzelle, ist es möglich, die Partikeln durch einen kleinen Stromfaden zu führen, der in eine Strömung durch eine größere Öffnung eingebettet ist (Hüllstrom). Geeignete Verfahren zur Erzielung eines Hüllstroms sind im Stand der Technik bekannt. Beispielsweise ist die Hüllstromtechnik aus der Anwendung in den aus dem Stand der Technik bekannten Zellen zur 2D Bildanalyse bekannt. Bei Verwendung einer koaxialen Hüllströmung, um den partikelführenden Strom in einen zentral im Strömungsquerschnitt liegenden Stromfaden auszuziehen, kann die Produktströmung ähnlich wie die trockene Dosierung nach jeder erfassten Partikel angehalten werden (während der Hüllstrom weiterläuft), um Zeit für die Speicherung und Auswertung zu gewinnen. Diese Varianten iia (regelmäßige Intervalle) und iic (particle on demand) sind mit rechnergesteuerten Pumpen und Ventilen realisierbar. Bei der flüssigen Förderung ist die Gefahr, dass sich Partikeln auf der Förderstrecke einholen, auch nicht so groß, so dass auch kleine Gruppen (2-5) unter Verwendung eines schnellen Zwischenspeichers verwendet werden können (vgl. Bilderfassung (Verfahrensschritt b))).

Grundsätzlich ist es möglich, eine Dosierung mit einem sehr geringem Partikelstrom durch einfache Einstellung der Fördergeschwindigkeit des Partikelstroms durchzuführen, wobei dieses Verfahren sowohl für pulverförmige Proben als auch für Proben, die in einer Dispersion vorliegen, geeignet ist.

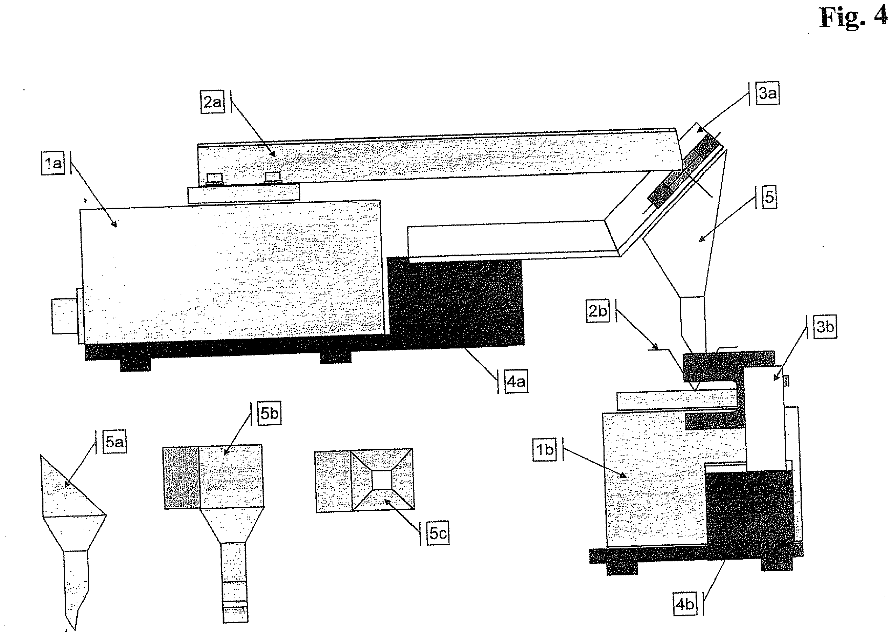

Um eine zuverlässige Vereinzelung der Partikel zu erhalten ist es jedoch bei pulverförmigen Proben bevorzugt, die Dosierung so auszuführen, dass jede Partikel einzeln auf die Förderstrecke abgegeben wird. Bevorzugt werden Dosierrinnen zur Dosierung von pulverförmigen Proben eingesetzt. Diese weisen in einer bevorzugten Ausführungsform einen V-förmigen Rinnenboden auf, der gegebenenfalls verrundet sein kann. Besonders bevorzugt werden zwei oder mehr hintereinander geschaltete Dosierrinnen eingesetzt. Die Dosierung der einzelnen Partikeln auf die Förderstrecke kann z.B. mit Hilfe von einer, bevorzugt zwei oder mehr hintereinander geschalteten, bevorzugt mit Lichtschranken geregelten Dosierrinnen erfolgen. Besonders bevorzugt ist eine Ausführung, bei der Rinnenachse, Schwerkraft und Lichtweg in einer Ebene liegen, und die Lichtschranke bevorzugt geneigt ist, zum Beispiel ca. 45 °. Somit unterbricht die Partikel die Lichtschranke stets kurz nach dem Ablösen von der Rinne, unabhängig von der Fluggeschwindigkeit. Für kleinere Partikeln unter 1 mm nochmals bevorzugt ist eine Verminderung der Verrundung des V-Rinnenbodens auf Radien zwischen 100 und 500$\mu$m, um diese kleinen Partikeln besser zu zentrieren. Geeignete Ausführungsformen für Lichtschranken und Dosierrinnen sind dem Fachmann bekannt. Insbesondere ist bekannt, dass die Detektionsgrenze einer Lichtschranke im Allgemeinen bei ca. 1/10 bis 1/30 des Querschnittes liegt, d.h. z.B. bei 30 bis 100 $\mu$m Durchmesser bei 1 mm Strahlquerschnitt. Ganz besonders bevorzugt werden die Partikeln zur Aufbringung auf die jeweils untenliegende Dosierrinne in einen Trichter geführt, damit sie sicher eingefangen und mit möglichst geringer Anfangsgeschwindigkeit und vorteilhafter Richtung (quer zur Förderung) auf die nachfolgende Dosierrinne oder Förderrinne bzw. Messküvette abgelegt werden. In Figur 4, Details 5, 5a, 5b, 5c ist eine bevorzugte Ausführungsform eines Trichters und dessen Anordnung bezüglich der Dosierrinnen dargestellt.

Wenn aus einer großen Probe nur ein kleiner Teil ausgewertet werden kann, dieser aber repräsentativ ausgewählt werden soll und eine vorangehende Probenteilung nicht erwünscht ist, kann man eine "in-line" Probenteilung in die Dosierung integrieren, und nur einen Bruchteil der Probe messen, und den Rest in einen separaten Auffangbehälter umleiten.

Da die Dosierung, insbesondere eine pulverförmige Dosierung, bevorzugt mehrstufig - mit Hilfe von zwei oder mehr hintereinander geschalteten Dosierrinnen - ausgeführt wird, bietet es sich an, nach der ersten Dosierung (aus dem Vorlagebehälter heraus in eine erste Dosierrinne) z.B. eine elektromechanische Klappe zu installieren, die ständig in programmierten Zeitverhältnissen zwischen Durchleitung und Ausschleusung umschaltet.

Eine geeignete Vorrichtung zur Dosierung von Partikeln pulverförmiger Proben ist in Figur 4 dargestellt.

Bei der nassen Förderung, d.h. in dem Fall, wenn die Probe in Form einer Dispersion vorliegt, wird im Allgemeinen eine Dispersion passender Konzentration hergestellt und durch eine geeignete Messküvette, die im Folgenden näher beschrieben wird, gepumpt (Kreis oder Passage). Die geeignete Konzentration (von hohen Konzentrationen kommend) ist dann gegeben, wenn keine Koinzidenzen mehr auftreten, und die Bilder qualitativ gut genug für die automatische Erkennung sind. Zu geringe Konzentrationen äußern sich in geringen Partikel-Zählraten. Im Allgemeinen erfolgen bei einem Vorliegen der Probe in Form einer Dispersion die vereinzelnde Dosierung durch die Verdünnung, die Förderung durch das Umpumpen und das Ausrichten durch die Formgebung im Einlauf der Messzelle. Auch durch einen Hüllstrom wird die Probe verdünnt.

*ab) Ausrichtung*

Erfindungsgemäß erfolgt die Ausrichtung der Längsachse der Partikeln in Förderrichtung, (vgl. "Förderung entlang einer Linie" in ac). Alle Partikelachsen werden bei der trockenen Förderung durch die zwei einwirkenden Anlageflächen ausgerichtet. Reibung und Schwerkraft bewirken die gewünschte Ausrichtung. Im Falle von Proben in Form einer flüssigen Dispersion wird unter einer Linie im Sinne der vorliegenden Anmeldung auch eine Stromlinie (Stromfaden) verstanden, der in einer Ausführungsform in einen Hüllstrom eingehüllt sein kann. Durch die erfindungsgemäße Ausrichtung kann in kurzer Zeit die individuelle dreidimensionale Form auch von komplex geformten Partikeln ermittelt werden. Aufgrund der Ausrichtung der Partikelachsen entlang einer Linie quer zur Beobachtungsrichtung kann eine maximale Menge an Informationen betreffend die Form der Teilchen mit einer minimalen Datenmenge (Anzahl Bilder/Beobachtungsrichtungen) erhalten werden, so dass eine Anwendung des erfindungsgemäßen Verfahrens z.B. in der Partikelmesstechnik möglich ist.

Die erfindungsgemäße definierte Ausrichtung der Partikeln ist von großer Bedeutung. Der einfachste vorstellbare Fall ist eine undefinierte und nicht bekannte Ausrichtung, z.B. im freien Fall oder beim Transport in einer Flüssigkeit ohne Ausrichtungszulauf. In diesem Fall ist eine genaue Formerfassung nicht möglich, außer in dem unrealistischen Fall, dass so viele Beobachtungsrichtungen erfasst werden, dass sich darunter auch die mit den gerade für die betrachtete Partikel günstigen Winkeln befinden. Wenn die Beobachtung quer zur Achse geringster Trägheit erfolgt, und entlang der beiden anderen, ist der Informationsgewinn maximal.

In einer bevorzugten Ausführungsform der vorliegenden Erfindung erfolgt eine Beobachtung der trocken an zwei Flächen ausgerichteten Partikeln aus zwei Beobachtungsrichtungen, im Allgemeinen mit Hilfe von zwei Kameras (siehe Verfahrensschritt b)).

In dieser Ausführungsform ist bevorzugt eine rechter Winkel der Anlageflächen und eine Beobachtung parallel zu den Anlageflächen (und quer zur Förderung gewählt).

In einer weiteren besonders bevorzugten Ausführungsform der vorliegenden Erfindung erfolgt eine Beobachtung der ausgerichteten Partikeln aus drei oder mehr, ganz besonders bevorzugt aus drei oder vier, insbesondere bevorzugt aus vier Beobachtungsrichtungen (im Allgemeinen mit Hilfe einer entsprechenden Zahl von Kameras) (siehe Verfahrensschritt b)).

In dieser Ausführungsform werden bei einer Ausrichtung der Partikeln in Längsachse ausreichend Informationen zur genauen Analyse der Partikeln erhalten. Man beachte, dass auch die weiteren Achsen durch die Anlageflächen ausgerichtet werden. Die Ausführungsform erfordert aber nicht, dass die Beobachtung nur parallel zu diesen Flächen erfolgt.

Die Verfahren und Vorrichtungen zur trockenen Ausrichtung nutzen demnach die zwei Anlageflächen und die Einwirkung von Schwerkraft und Reibung, wie sie durch die Wahl der Förderparameter eingestellt werden. Bei der trockenen Förderung ist demnach eine Ausrichtung in allen Achsen die Regel, denn um die Achse geringster Trägheit auszurichten, werden die anderen in der Regel mit ausgerichtet. Bei Anlage an nur eine Fläche wird dagegen nur die Achse größter Trägheit senkrecht zu dieser Fläche ausgerichtet ("stabile Lage").

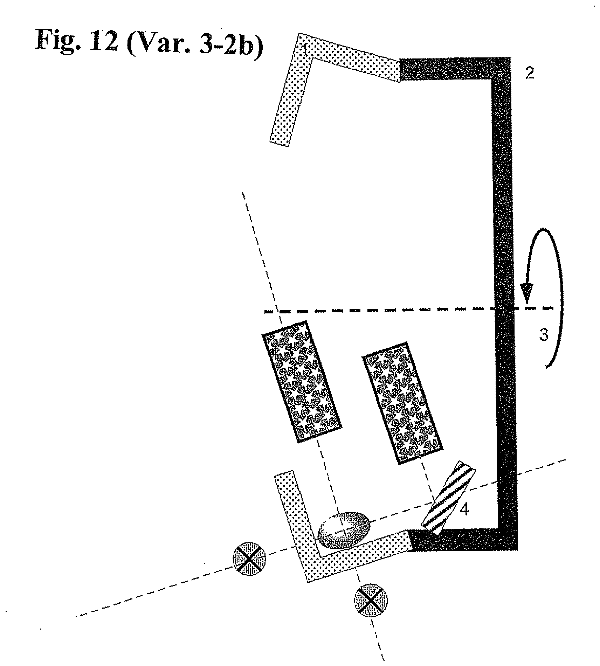

In trockener Umgebung (im Allgemeinen bei Proben in Pulverform) haben die Partikeln in der Regel Kontakt mit zwei planen Flächen (Anlageflächen, Küvettenwände), wenn sie von Schwerkraft oder Zentrifugalkraft in Richtung der Schnittlinie dieser Flächen gezogen werden. Dabei werden Sie ausgerichtet, um den Zustand geringster Energie zu erreichen. Dabei werden die Partikeln bevorzugt entlang der Schnittlinie der zwei Flächen gefördert. Diese beiden Anlageflächen bilden die Förderrinne (alternativ ist auch eine gekrümmte Rinne möglich, z.B. mit Kreis-, Hyperbel -oder Parabel-Profil). Neben der Schwerkraft können auch zentrifugale Kräfte eingesetzt werden (siehe Variante 3-2b, Figur 12). Die optimale, d.h. ganz besonders bevorzugte, Beobachtungsrichtung verläuft tangential zu den ausrichtenden Anlageflächen oder Küvettenwänden. Bevorzugte geeignete Ausführungsformen betreffend die optische Realisierung sind nachstehend beschrieben.

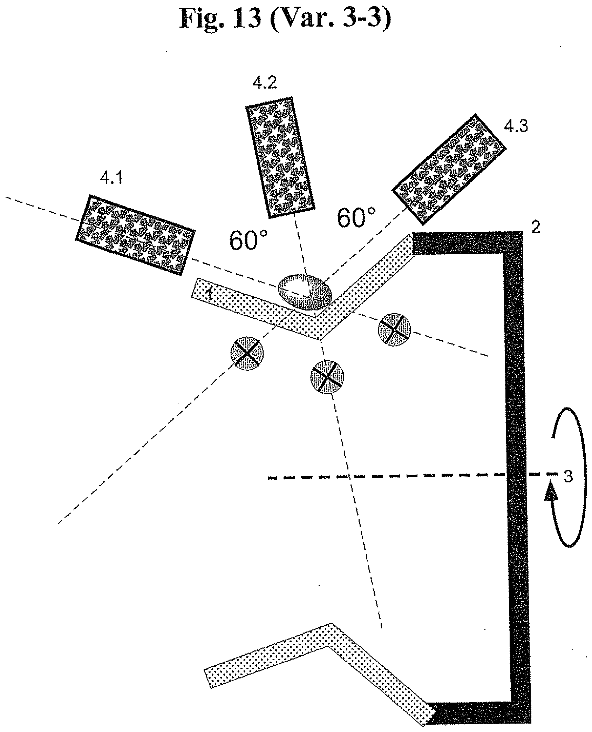

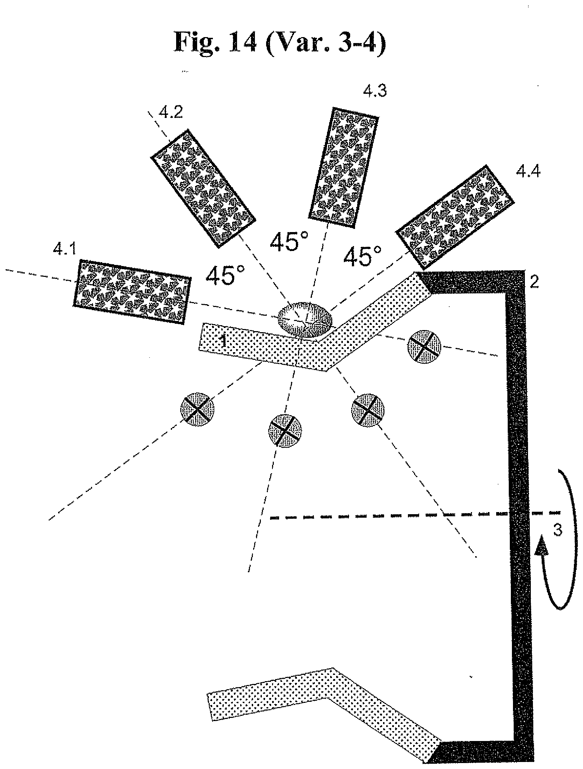

Für die Form der Anlageflächen oder Küvettenwände gibt es verschiedene Möglichkeiten. Gut geeignet sind Präzisionsküvetten (daher auch die Bezeichnung Küvettenwände für die Anlageflächen), die bevorzugt quadratisch oder rechteckig sind, oder V-Rinnen, bevorzugt 90° V-Rinnen, die z.B. durch einseitiges Abschleifen solcher Präzisionsküvetten hergestellt werden können. Im Beobachtungsteil sind die freien Kanten bevorzugt senkrecht zu den Küvettenflächen angeschliffen, dann erscheinen diese Bereiche im Bild hell. Bei anderen Winkeln, z.B. 45°, erscheinen diese Bereiche dunkel. Alternativ erfolgt nur ein Ausbruch im Beobachtungsteil, so dass die Küvette in Teilbereichen geschlossen bleibt. Es können für die Anordnung der Küvettenwände oder V-Rinnen auch andere Winkel als 90° gewählt werden. Dabei ist zu beachten, dass diese Flächen die Beleuchtung und Beobachtung nicht stören dürfen. Bevorzugt sollte es bei 2 Winkeln (= zwei Beobachtungsrichtungen) möglich sein, die Partikel tangential zur jeweiligen Anlagefläche zu beobachten, damit man die Vorteile dieser informativsten Richtung ausnutzen kann. Die weiteren, zusätzlichen Winkel (= Beobachtungsrichtungen) werden weiter unten dargestellt. Besonders bevorzugt sind in Beobachtungsrichtung so wenig Kanten, schräge oder raue Flächen wie möglich vorhanden, da diese störend sein können. Die Varianten 3-3 (siehe Figur 13) und 3-4 (siehe Figur 14) illustrieren Winkel von 120° = 2 × 60° (3 Beobachtungsrichtungen) und 135°= 3 × 45° (4 Beobachtungsrichtungen).

Die Förderrinnen, bevorzugt Präzisionsküvetten oder V-Rinnen, müssen keine planparallelen Seiten aufweisen. Die Innen-Flächen, bzw. der Knick zwischen zwei Flächen, gegen die die Partikel durch die Schwerkraft oder Zentrifugalkraft zentriert und ausgerichtet wird, können unabhängig von der Außenfläche gestaltet werden. Gemeinsame Randbedingung für alle Ausführungen ist, dass die Objektive und ihre Strahlengänge Zugang zu den betrachteten Partikeln haben müssen.

Die Ausrichtung von Partikeln von Proben in Pulverform erfolgt somit gegen zwei plane Flächen, die eine Förderrinne bilden, mit Hilfe der Schwerkraft oder mit Hilfe von Zentrifugalkräften.

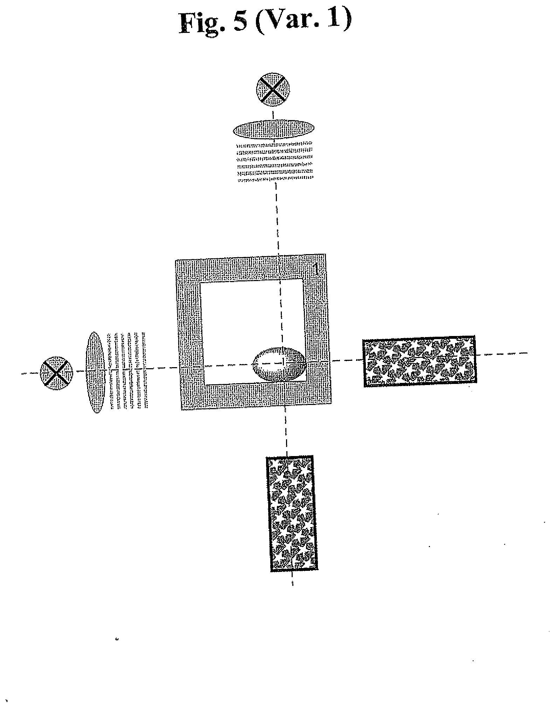

Beispiele für geeignete Förderrinnen und dazu passende Beleuchtungs- und Beobachtungssysteme sind in den Figuren 5 bis 18 dargestellt, worin bevorzugte Ausführungsformen der vorliegenden Erfindung dargestellt sind. Der Einsatz der in diesen Figuren dargestellten Förderrinnen ist unabhängig von der in den Figuren dargestellten speziellen Ausführungsform.

Die vorstehenden Ausführungen betreffend die Ausrichtung von Partikeln von Proben in Pulverform mit Hilfe von Förderrinnen betreffen eine bevorzugte mehrachsige Ausrichtung der Partikeln. Wie vorstehend erwähnt, ist in dem erfindungsgemäßen Verfahren bzw. der erfindungsgemäßen Vorrichtung immer eine Ausrichtung der Achse gerinster Trägheit parallel zur Förderrichtung und quer zur Beobachtung angestrebt. Für zwei Beobachtungsrichtungen sollten auch die beiden anderen Achsen parallel zur Beobachtung ausgerichtet sein, bei einer Beobachtung aus drei oder mehr Beobachtungsrichtungen ist dies weniger wichtig. Man beachte, dass die Annäherung an die Flächenschnittlinie bzw. allgemein die Linie geringster Lageenergie (s.o.) auch bewirkt, dass die Partikeln in die Förderlinie zentriert und damit in den Schärfenbereicht der Beobachtungen gebracht werden, was ein beabsichtigter und notwendiger Effekt ist.

Bevorzugt erfolgt die erfindungsgemäße Ausrichtung und Zentrierung der Partikeln durch die Schwerkraft. Je nach Neigung des Profils (der Förderrinne) kann eine Anlagefläche durch eine größere Normalkomponente bevorzugt werden. Als vorteilhaft bewährt haben sich Neigungswinkel zwischen 15° und 35° Bei Beobachtung unter 3 oder mehr Winkeln in einer Ebene ist die zusätzliche Ausrichtung um die Längsachse (mehrachsige Ausrichtung) nicht mehr so wichtig, nur die Ausrichtung in Längsrichtung. Eine Bevorzugung einer Anlagefläche ist dann nicht mehr nötig, aber eine Neigung in Richtung des Knickes ist für alle Flächen zur Sicherstellung der Ausrichtung und Zentrierung erforderlich. Die Längsausrichtung ist selbst in einer relativ flachen Nut mit mehr als 90° gegeben, also z.B. bei 120° oder 135°, hier sind dann beide Ausrichtungs-Winkel allerdings sehr flach, und sollten bevorzugt gleich gewählt werden.

Die Ausrichtung von Partikeln von Proben in Form von Dispersionen erfolgt bevorzugt durch Strömungskräfte in einer Strömungszelle. Eine bevorzugt eingesetzte Strömungszelle zur zweiachsigen Ausrichtung von Partikeln, die in einer Dispersion vorliegen, ist in der PCT-Anmeldung PCT/EP/2004/014603 mit dem Titel "Hochgenauer strömungsorientierter Mehrwinkel-Remissionssensor" offenbart, und wird nachstehend näher ausgeführt (siehe ac)).

Ähnlich wie bei der trockenen Förderung ist die Beobachtung unter 4 Winkeln in einer oktagonalen Durchflusszelle bevorzugt, besonders bevorzugt in Verbindung mit einem Hüllstrom zur Zentrierung der Partikeln in einem Stromfaden.

*ac) automatisierte Förderung*

Der Begriff Förderung wird hier für den Abschnitt des Partikel-Transportes verwendet, der die Partikel durch das Beobachtungsvolumen befördert. Wie bereits vorstehend ausgeführt, kann die Förderung der Partikeln gleichzeitig mit der Ausrichtung der Partikeln sowie gegebenenfalls zusätzlich gleichzeitig mit der Dosierung erfolgen.

Bei der nassen Förderung (z.B. von Dispersionen) wird die Probe bevorzugt durch die Strömung gefördert (z.B. mittels Pumpe oder Druckgefälle). Auslegungsüberlegungen finden sich weiter unten.

Bei der trockenen Förderung kann man die Probe (z.B. in Pulverform) entlang der Schnittlinie von zwei eine Förderrinne bildenden Flächen entlang einer Linie (zum Beispiel in einer schwingenden Förderrinne) gleiten lassen (Rutschbetrieb) (Hangabtriebskraft und/oder Vibrationsförderung) oder auf einer bewegten Schnittlinie von zwei eine Förderrinne bildenden Flächen absetzen (mitlaufende, z.B. rotierende Förderrinne) und später wieder entfernen (mitlaufende Förderung).

Für die Förderung im Rutschbetrieb mittels einer schwingenden Förderrinne stehen drei Parameter zur Verfügung, die kombiniert werden können. Zum einen der Hangabtrieb und zum anderen die Vibration der Rinne mit Hub und Längsbewegung. Bei runden Partikeln, die zum Rollen neigen, wird der Hangabtrieb bevorzugt gering gewählt. Die Vibration wird eingestellt durch den Vibrationswinkel, die Frequenz und die Amplitude. Eine bevorzugte Möglichkeit, die Förderrinne schwingfähig (vibrationsfähig) aufzuhängen, ist eine doppelte Federbandführung mit einer Neigung der Federbänder, damit die Anordnung einen Hub ausführt. Bei senkrecht stehenden Bändern ist der Hub dagegen null. Als Antrieb kann z.B. ein Elektromagnet eingesetzt werden, z.B. ein kompakter, langhubiger Basslautsprecher, an dessen Schwingspule eine Schubstange befestigt wird, die die Förderrinne antreibt. Ein Frequenzgenerator (z.B. Einzelgerät, oder D/A-Karte im PC) mit Leistungsverstärker treibt in einer bevorzugten Ausführungsform den Lautsprecher mit einer einstellbaren Frequenz (im Normalfall die Resonanzfrequenz der Anordnung) und Amplitude an (diese Einstellungs- und Ausführungsmöglichkeiten sind dem Fachmann bekannt und können zum Teil von handelsüblichen Dosiergeräten übernommen werden). Bei Antrieb mit Resonanzfrequenz ergibt sich immer eine sinusförmige Bewegung, aber bei entsprechender Antriebskraft kann man im nichtresonanten Betrieb auch andere Bewegungsformen erzwingen. Die Gleitförderung wird in der Regel durch Anpassung der Neigungswinkel und des Hubes (Winkel und Amplitude der Förderung) auf das Produkt eingestellt. Dafür kann man z.B. die Gleitfläche aus relativ kurzen, geraden Teilflächen zusammensetzen, z.B. aus gut verfügbaren optisch einwandfreien Küvetten. In der Regel sind in der Ebene senkrecht zur Förderrichtungen die Komponenten von Hub- und Schwerkraft parallel, andere Winkel sind möglich, bringen aber keine Vorteile.

Die Förderrinne, die für den Rutschbetrieb eingesetzt wird, hat somit im Allgemeinen einen Neigungswinkel in Längsrichtung (Förderrichtung) von 0 bis 25°. Allgemein bevorzugte Winkel gibt es nicht, da dies ein produktabhängiger Parameter ist, der experimentell ermittelt werden muss. Ein bewährter Wert, um diese Anpassung zu beginnen liegt zwischen 7 und 10°.

In Querrichtung (senkrecht zur Förderrichtung) weist die Förderrinne im Allgemeinen einen Neigungswinkel von 0 bis 45 °, auf. Bei Systemen mit 2 Beobachtungsrichtungen ist der bevorzugte Bereich 15 bis 35 °, bei Systemen mit 4 Beobachtungsrichtungen bevorzugt 45°, da dies am einfachsten zu justieren ist.

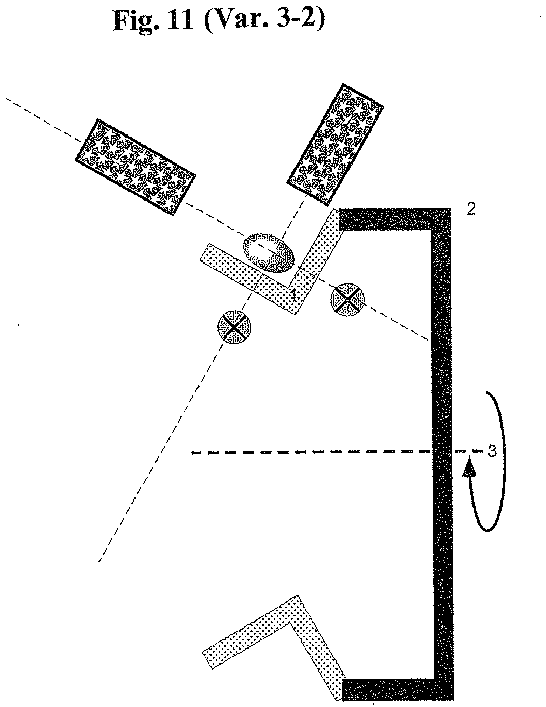

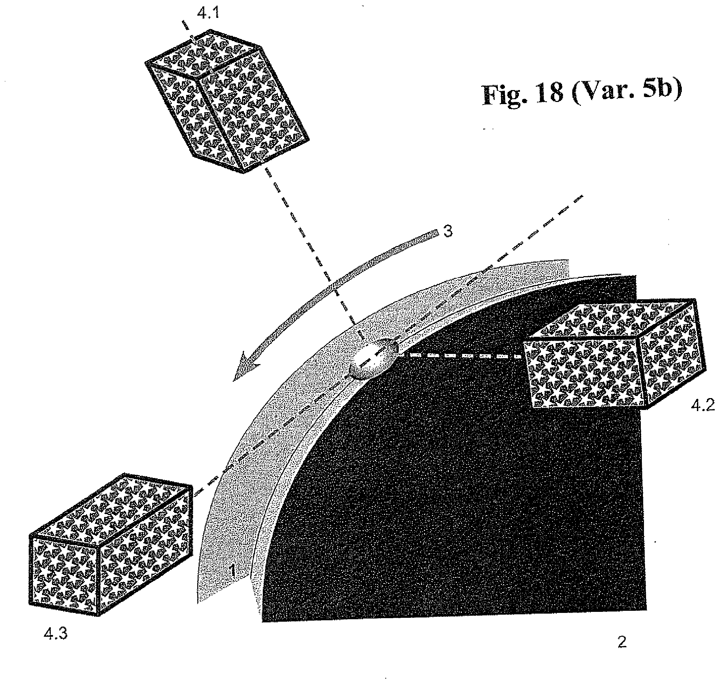

Mitlaufende Förderungen sind zwar sehr attraktiv, sie müssen aber in Förderrichtung kreis- bzw. ringförmig ausgeführt werden, oder als flexibles Band (vgl. Bandsäge). Dies so zu gestalten, dass sich am Beobachtungsort trotzdem die benötigten optischen Eigenschaften ergeben, ist sehr aufwändig. Konkrete Ausführungsführungsformen betreffend eine mitlaufende Förderung sind nachstehend beschrieben (Variante 3, siehe Figuren 11 bis 15, Variante 5b, siehe Figur 18). Der Einsatz der in diesen Figuren dargestellten Förderrinnen betreffend eine mitlaufende Förderung ist unabhängig von den in den Figuren dargestellten speziellen Ausführungsformen.

Nach der Beobachtung der ausgerichteten Partikeln werden diese in der Regel aus der Förderrinne entfernt. Bei den meisten Ausführungsformen fällt die Partikel nach der Förderstrecke (am Ende der Förderrinne) "von selbst" in einen Auffangbehälter, was für ein nachträgliches Wiegen von Vorteil ist. Ansonsten, z.B. bei mitlaufenden Förderungen, kann man die Partikeln abstreifen, abbürsten oder absaugen, z.B. bei Förderung im Zentrifugalfeld (Variante 3-2b, Figur 12).

Schließlich ist noch der Einfuß des Produktes auf die Produktförderung zu bedenken. Viele Produkte sind noch bei Partikelgrößen von z.B. 50 $\mu$m rieselfähig und sind damit mit einer Rinne förderbar. Die Grenze hängt von der Oberflächenbeschaffenheit ab, der Form und der Dichte. Die Zusammenhänge sind dem Fachmann bekannt, da das Handeln von Produkten eine Standardaufgabe der Verfahrenstechnik ist. Sehr leichte oder gar klebrige Produkte, die nicht gut rieselfähig sind, sind z.B. mit einer Vorrichtung gemäß Variante 3 und 5 (siehe Figuren 11 bis 15 und 18) förderbar.

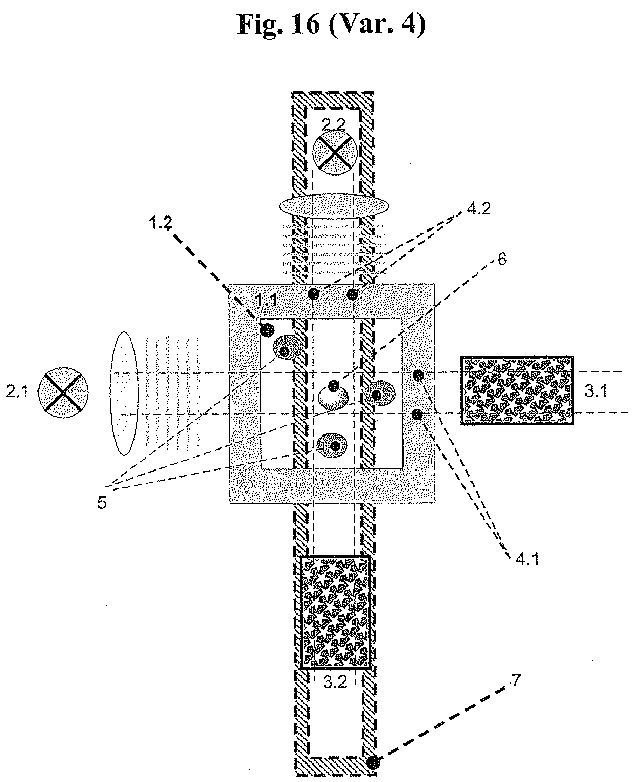

Die Maße der Produktförderrinnen und die Vergrößerungsmaßstäbe können an die Partikelgröße angepasst werden. Prinzipiell gibt es keine obere Grenze und als untere Grenze kann man für die trockene Förderung (Probe in Form von Pulver) je nach Produkt 20 bis 300 $\mu$m ansehen. Bei der nassen Förderung (Variante 4, siehe Figur 16) in Form von Dispersionen, die sich für die Handhabung von nicht rieselfähigen Partikeln anbietet, kann man Partikel ab etwa 2 $\mu$m erkennen, und ab etwa 5 $\mu$m sinnvoll erste Forminformationen gewinnen. Mit Hilfe des erfindungsgemäßen Verfahrens bzw. der erfindungsgemäßen Vorrichtung kann somit die individuelle dreidimensionale Form von Partikeln mit verschiedenen Teilchengrößen ab einer Teilchengröße von etwa 5 $\square$m ermittelt werden.

Als Materialien für die Förderrinnen (trockene Förderung) sind verschiedene Materialien einsetzbar. Wichtig ist, dass eine Beobachtung der Partikeln möglich ist. Bei Einsatz einer "geschlossenen" Förderrinne, z.B. in Form einer Küvette oder eines Rohres ist der Einsatz von transparenten Materialien wie Glas, Polymer, Keramik, Saphir, Diamant bevorzugt. Bei Einsatz von "offenen" Förderrinnen, z.B. V-Rinnen, können neben den transparenten Materialien, die vorstehend erwähnt sind, weitere Materialien eingesetzt werden, z.B. weiße, diffus streuende Materialien (z.B. Milchglas, weißer Kunststoff, Teflon, Keramik), Spiegel, sowohl 100%-Spiegel als auch halbdurchlässige Spiegel sowie undurchsichtige Materialien wie Metalle. Geeignete Materialien, die die vorstehenden Eigenschaften aufweisen und als Förderrinnen in dem erfindungsgemäßen Verfahren bzw. der erfindungsgemäßen Vorrichtung eingesetzt werden können, sind dem Fachmann bekannt. Im Folgenden werden weitere Ausführungen zu für bestimmte Ausführungsformen bevorzugten Materialien gemacht (siehe bac)).

Die automatisierte Förderung von Partikeln von Proben, die in Form einer Dispersion vorliegen, die in einer Strömungszelle erfolgt, erfolgt bevorzugt mittels einer Pumpe oder eines Druckgefälles.

Eine üblicherweise zur Ausrichtung und Messung der Partikeln von Proben, die in Dispersion vorliegen, eingesetzte Strömungszelle endet im Allgemeinen in einer Küvette. Die Küvette zur Messung der ausgerichteten Partikeln kann quadratisch oder 6- oder 8-eckig sein. Welche diese Ausführungen zu bevorzugen ist, hängt im Wesentlichen von der Anwendung ab. Verfügbarkeit/Preis, optische Auflösung, Ausrichtungsaufwand und Genauigkeit der Volumen- und Formerfassung müssen abgewogen werden. Soweit nicht oben beschrieben, sind diese Abschätzungen dem Fachmann bekannt (z.B. Auflösungsvermögen). Zu beachten ist, dass diese transparenten Küvetten an allen Flächen optische Qualität und unsichtbare Fugen aufweisen müssen (eine geeignete Fügetechnik ist im Stand der Technik bekannt, z.B. von Hellma)

Als Material für Küvetten sind transparente Materialien, insbesondere Glas und die anderen oben genannten geeignet.

Das Verhältnis aus Schärfentiefebereich und Querschnitt der Küvette bei der nassen Förderung sollte bevorzugt nicht kleiner als 1:10 sein. Diese Randbedingung entfällt bei Verwendung eines Hüllstroms. Handelsübliche quadratische Küvetten in optischer Qualität (Präzisionsküvetten) weisen einen minimalen Querschnitt von etwa 1mm*1mm auf. Küvetten mit kleineren Querschnitten und Wandstärken sind - bei entsprechender Verfügbarkeit ebenfalls vorteilhaft (z.B. 0.5 oder 0.2 mm).

Die eingesetzte - vorstehend erwähnte - Messzelle, die bei der Untersuchung von Proben in Form von Dispersionen bevorzugt eingesetzt wird (Strömungszelle), ermöglicht eine zweidimensionale Ausrichtung der Partikel der Probe und ist z. B. in der PCT-Anmeldung PCT/EP/2004/014603 mit dem Titel "Hochgenauer strömungsorientierter Mehrwinkel-Remissionssensor" offenbart. Dabei handelt es sich um eine dreidimensionale Strömungszelle zur Ausrichtung von nicht-isometrischen Partikeln in einer flüssigen Probe in zwei Achsen, umfassend eine Zulaufzone für die Probe enthaltend auszurichtende Partikel und einen Auslass für die Probe enthaltend in zwei Achsen ausgerichtete Partikel, wobei ein Fluidelement der Probe mit den Maßen a, b, c in einer Dehnungszone zu einem Fluidelement mit den Maßen a $\times$

n, b/(n × m), c × m umgeformt wird, wobei a die Breite, b die Höhe und c die Länge des Fluidelements bedeuten, und n und m von der Geometrie der Strömungszelle abhängige Konstanten (Dehnungsgrad) sind, die positive Zahlen ≥ 1 bedeuten. Auf diese Situation angewendet, muss im Strömungsteil der Querschnitt in eine Richtung gedehnt werden, so dass der Eintrittsspalt um dieses Verhältnis enger wird als der Küvettenquerschnitt. Sehr enge Querschnitte sind schwer zu fertigen, und neigen bei einzelnen zu großen Partikeln auch zum Verstopfen. Für den Zweck der 3D-Form-Messung reichen allerdings auch schon Querdehnungen von 1.5, 1.8 oder 2.0 aus. Die Längsdehnung ist unkritisch auszuführen und kann von 2 bis 5 gewählt werden.

Wie bereits vorstehend erwähnt, ist anstelle der Führung einer Dispersion enthaltend die zu messenden Partikeln durch die Messzelle die Führung der Partikeln durch einen kleinen Stromfaden, der in eine Strömung durch eine größere Öffnung eingebettet ist, möglich (Hüllstrom). Im Allgemeinen wird mit Hilfe des genannten Stromfadens eine einachsige Ausrichtung erzielt. In einer Ausführungsform kann ein Hüllstrom daher dann eingesetzt werden, wenn eine Beobachtung der ausgerichteten Partikeln aus drei oder mehr Beobachtungsrichtungen erfolgt. Die Nutzung eines Hüllstroms ist bei Einsatz aller vorstehend genannten Küvetten grundsätzlich möglich.

*b) Beobachtung der ausgerichteten Partikel mit Hilfe von mindestens zwei Kameras und Bilderfassung, wobei eine Aufzeichnung aller Bilder, auf denen Partikel zumindest teilweise abgebildet sind, erfolgt*

Nach der Bilderfassung ist es möglich, einen ausgewählten Bildersatz einer Partikel direkt zu verarbeiten, d. h. auszuwerten, und anschließend die nächste Partikel zu messen, oder einen ausgewählten Bildersatz einer Partikel zunächst ohne weitere Auswertung zu speichern und nach Speichern die nächste Partikel zu messen. Die Auswertung erfolgt dann zum Beispiel im Anschluss an die Messung und Speicherung der entsprechenden Bildersätze aller Partikeln einer Probe. In einer bevorzugten Ausführungsform erfolgt eine direkte Verarbeitung des Bildersatzes einer Partikel, bevor das nächste Partikel zur Messung "angefordert" wird ("particle on demand").

*ba) Beobachtung*

*baa) Anzahl verwendeter Beobachtungsrichtungen*

Jede Beobachtungsrichtung begrenzt durch die von ihr wahrgenommene Projektionsfläche das maximale Volumen nach oben, das die beobachtete Partikel einnehmen kann, sowie das kleinste Volumen nach unten. Ist die Anzahl 1, so handelt es sich um eine aus dem Stand der Technik bekannte 2D Formbeschreibung. Diese Ausführung begrenzt das Volumen nicht, da sie in der Tiefe keine Information liefert. Eine Volumenbestimmung ist bei der 2D Formenbeschreibung nur für Kugeln möglich.

Gemäß der vorliegenden Erfindung beträgt die Zahl der Beobachtungsrichtungen mindestens 2, bevorzugt 2, 3 oder 4, besonders bevorzugt 4 (Varianten 2, 2b, 2c). Alle Ausführungen mit 2, 3, 4 oder mehr Beobachtungsrichtungen führen zu einem begrenzten Volumen. Im Allgemeinen entspricht die Zahl der Kameras der Zahl der Beobachtungsrichtungen. Man kann nun vergleichen, welche die größte und kleinste konvexe Form ist, die zu den Projektionsflächen passt. Je geringer der Unterschied, desto geringer ist der maximal mögliche Fehler bei der Volumenbestimmung. Durch eine geschickte Ausrichtung der Partikeln, wie sie erfindungsgemäß erfolgt, lässt sich der Fehler stark reduzieren.

*bab) Winkel zwischen den Beobachtungsrichtungen*

Werden zwei Kameras eingesetzt, d.h, beträgt die Zahl der Beobachtungsrichtungen 2, beträgt der Winkel zwischen der Beobachtungsrichtungen bevorzugt 90°, da dies der Winkel mit dem höchsten Informationsgewinn ist. Bevorzugt sind die Kameras bei Einsatz von zwei Kameras somit orthogonal zueinander ausgerichtet.

Bei drei oder mehr Richtungen können die Beobachtungsrichtungen jeweils orthogonal zueinander sein. Dies ist jedoch nicht generell die besonders bevorzugte Anordnung (siehe Variante 5, 5b). Je nach Ausrichtung der Partikeln können auch andere Winkel bevorzugt sein, z.B. 4 in 45°-Schritten in einer Ebene senkrecht zur Transportrichtung (Förderrichtung) angeordnete Beobachtungen (Varianten 2, 2b, 2c). In einer bevorzugten Ausführungsform liegen die Winkel der Beobachtungsrichtungen bei 3 oder mehr, bevorzugt 3 oder 4 Beobachtungsrichtungen in einer Ebene senkrecht zur Förderrichtung der Partikeln.

Jede Beobachtungsrichtung hat eine Ebene optimaler Schärfe, sowie einen parallel zu dieser Ebene liegenden Schärfentiefenbereich. Liegen alle Beobachtungsrichtungen in einer Ebene, so ergibt sich aus dem Schnitt der Schärfeebenen eine Linie, bzw. ein Schlauch der Schärfentiefe um diese Linie. Bei 3 und mehr Beobachtungsrichtungen, die nicht in einer Ebene liegen, reduziert sich die optimale Schärfe auf einen Punkt, bzw. auf ein kleines Volumen um diesen Punkt. Die Schärfentiefe ist, wie dem Fachmann bekannt ist, von der Auflösung der Abbildung abhängig, und liegt bei kleineren Partikeln nur noch in der Größenordnung der Partikelgröße. Dies bedeutet, dass im allgemeinen Fall nur noch Partikel entlang einer Linie ("Perlenschnur") oder in genau einem Punkt erfasst werden können. Die erfindungsgemäße Ausrichtung der Partikeln (siehe Schritt ab)) bewirkt nicht nur eine Orientierung der Achsen der Partikeln in günstige Richtungen, sie bewirkt auch eine Zentrierung der Partikeln in eine definierte Lage, so dass Ihr Weg der Schärfenlinie folgt bzw. durch den Schärfenpunkt verläuft. Dies ist von großem Vorteil, da man so auf einen zeitaufwendigen Autofokus verzichten kann. Die Ausführung mit einer Schärfenlinie ist besonders vorteilhaft, da sie in der Regel auf ein Anhalten der Bewegung oder auf ein Triggern der Bilderfassung (s.u.) verzichten kann.

*bac) Art der optischen Abbildung*

Es sind verschiedene Arten der optischen Abbildung möglich, z.B. Extinktionsdurchlicht (EDL = gerichtete Beleuchtung), diffuses Durchlicht (DDL), koaxiales Auflicht (KAL, meist mit Polarisation) oder konzentrisches Auflicht ZAL).

Die Art der optischen Abbildung unterscheidet sich zunächst wie folgt: Extinktionsdurchlicht (EDL = gerichtete Beleuchtung), bekannt aus Anordnungen mit telezentrischen Objektiven, die eine ebene Beleuchtungswelle verwenden. Für einfache Objektive mit divergenter Beobachtung (die man gegebenenfalls aus Platz- oder Kostengründen bevorzugt) kann man eine konvergente gerichtete Beleuchtung einrichten, die nahezu gleichwertige Ergebnisse liefert. Die weiteren Möglichkeiten sind diffuses Durchlicht (DDL), koaxiales Auflicht (KAL, meist mit Polarisation) oder konzentrisches Auflicht ZAL), wie bereits vorstehend erwähnt. Die Vor- und Nachteile dieser Abbildungsvarianten sind dem Fachmann generell bekannt (Vgl. "1D und 2D Abbildende Prozesssonden: Theorie und Praxis", Vortrag von Dr. M. Schäfer auf dem Fachausschuss Partikelmesstechnik am 23.02.2005 in Würzburg). Bei der Nutzung für mehrachsige Abbildung und automatische Auswertung ist zu beachten, dass die Abbildung eine automatische Erkennung der Partikelfläche ermöglichen muss. Dies bedeutet, dass zum einen eine eindeutige Unterscheidung zwischen Hintergrund und Partikelprojektionsfläche möglich sein muss. Bei der Realisierung in EDL und DDL erscheint die Partikel dunkel vor hellem Grund, besonders kontrastreich mit EDL. Bei transparenten Partikeln können Teile der Projektionsfläche hell erscheinen, bei EDL ist aber die Kontur stets geschlossen und die hellen Bereiche sind klein, so dass die Projektionsfläche mit den dem Fachmann bekannten Algorithmen für das Füllen von Löchern gefüllt werden können. Zu berücksichtigen ist, dass bei EDL auf jeden Fall die für die Ausrichtung der Partikeln oder die für die Führung der Flüssigkeits-Strömung genutzten Flächen durchstrahlt werden müssen.

Beobachtung und Beleuchtung stehen sich gegenüber, es ist zunächst gleich, ob die Beobachtung über oder unter der transparenten Führungsebene angebracht wird. Bei DDL kann die Auflagefläche selbst transparent sein, und von dem diffusen Licht durchstrahlt werden, oder sie ist aus einem streuenden Material passender Stärke (z.B. Milchglas, weißer Kunststoff, Teflon) aufgebaut, das hintergrundbeleuchtet wird. Die Beobachtung muss nicht notwendigerweise senkrecht zur durchstrahlten Glasfläche erfolgen. Bei ZAL und KAL wählt man in der Regel einen dunklen Hintergrund, vor dem sich die Partikeln hell abheben. Bei dunklen Partikeln kann man auch einen hellen Hintergrund wählen, sollte aber dann bevorzugt so beleuchten, dass keine Schatten entstehen. Alle AL Anordnungen können Probleme mit transparenten Partikeln haben, d.h., bevorzugt werden bei transparenten Partikeln andere Anordnungen gewählt. Bei KAL kann man aber auch einen Spiegel als Hintergrund nehmen, exakt senkrechte Beobachtung ohne Polarisation, und erhält ein "pseudo-EDL_Bild" mit ähnlichen Eigenschaften, insbesondere bezüglich transparenter Partikeln.

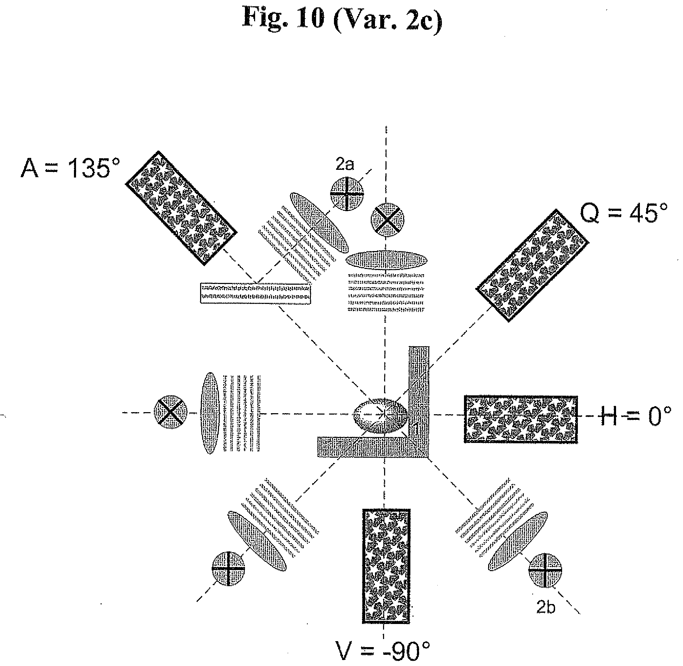

Eine Messung der Farbe der Partikeln erfolgt in der Regel im Auflicht. Daher bieten sich Beobachtungsrichtungen an, die nicht senkrecht auf die Förderrinne ausgerichtet sind (z.B. 135° oder 45°, siehe Variante 2b und 2c, Figur 10), und den spiegelnden Reflex vermeiden.

Bei allen Anordnungen ist zu beachten, dass die für eine Beobachtungsrichtung zuständige Beleuchtung eine andere Beobachtung nicht stören sollte. Eine Beleuchtung aus vielen Richtungen ist im Falle von Auflicht günstig, da sie Schatten vermeidet. Eine diffuse Beleuchtung aus vielen Richtungen kann dagegen weiße Partikeln so aufhellen, dass sie kaum noch zu erkennen sind.

Besonders bevorzugt wird daher EDL angewendet. Bei Küvetten als Förderrinnen ist die optische Qualität kein Problem, Profile für Drehzylinder, Bänder und Rinnen können dagegen nicht so ohne weiteres in optischer Qualität gefertigt werden. Diese Einschränkung kann man jedoch mildern, wenn man nur die Beleuchtung durch die verwendeten Materialien führt (z.B. Kunststoffe, Folien, warm verformte Gläser, transparente Keramik), und die Beobachtung auf die Partikelseite legt (bei Verwendung einer Förderrinne, die offen ist, d.h. z.B. ein V-Profil aufweist).

*bad) Kamera und Lichtquelle*

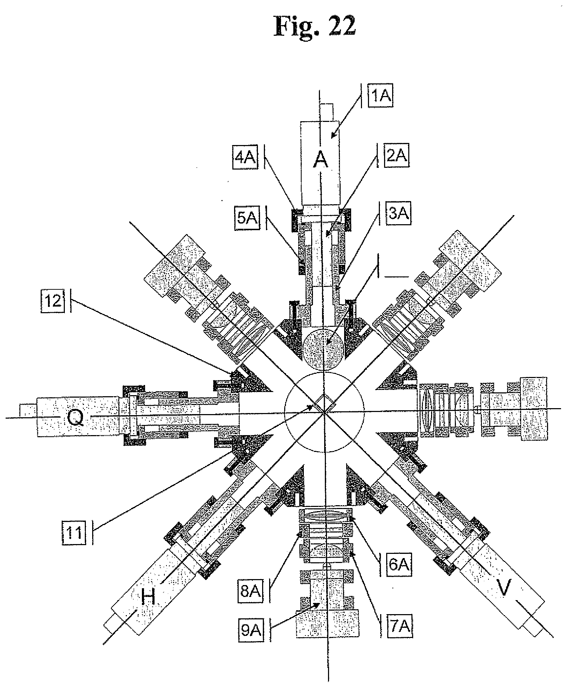

Die konkrete Wahl von Kamera und Lichtquelle hängt von der Art der Beleuchtung ab. Im Allgemeinen, insbesondere bei Durchlicht und allen Anordnungen mit Zentrierung (z.B. alle nachstehend genannten bevorzugten Ausführungsformen (alle Varianten) außer Variante 4, Figur 22, siehe unten), werden eine triggerbare S/W Kamera mit VGA-Auflösung, mit analoger Bildübermittlung (bevorzugt: progressive scan), ein mehrkanaliger Framegrabber mit z.B. 4 simultanen Kanälen, und einer Beleuchtung mit Kaltlichtquelle, Weißlicht-LED (für Durchlicht kommen auch farbige LEDs in Frage, für bestimmte Materialien sogar vorteilhaft) oder Halogenlampe eingesetzt. Vorteilhaft sind wegen der kompakten Bauform und der Vibrationsunempfindlichkeit Weißlicht-LEDs, z.B. 1W-Lumileds von Luxeon. Es sind aber vom Fachmann zahlreiche andere Varianten realisierbar, die ebenfalls einsetzbar sind, z.B. USB-Kameras, Kameras mit Digitalausgang, Cameralink, Fire-Wire, usw.

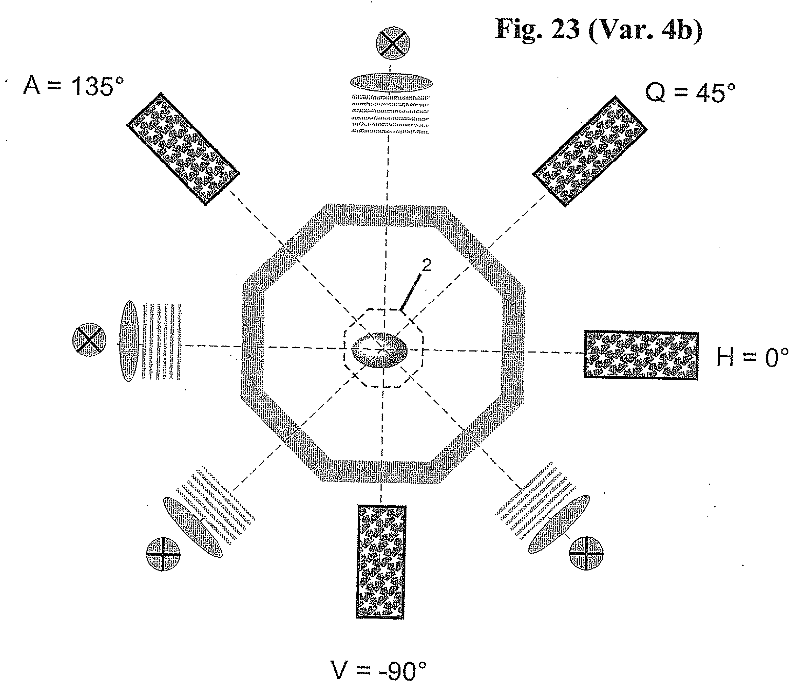

Die Kameras können in CCD und CMOS Technik hergestellt sein, die geringere Lichtstärke von CMOS ist bei Durchlicht kein Problem, außer bei hohen Auflösungen, wenn die Shutterzeit nicht mehr für die Reduktion der Bewegungsunschärfe ausreicht (der Zusammenhang zwischen Bewegungsunschärfe und Belichtungszeit ist dem Fachmann bekannt), dann kann man zum einen CCDs nehmen, stärkere Lichtquellen (z.B. 3W oder 5W Dioden, Kaltlichtquellen), oder Blitze (z.B. Wotan von Polytec). Bei Auflichtbildern muss in der Regel eine empfindlichere Kamera und/oder eine hellere Lichtquelle eingesetzt werden. Farbkameras sind nur dann sinnvoll, wenn ein Auflichtbild mit Farbinformation benötigt wird. Eine sinnvolle Kombination bei 4 Bildern sind dann z.B. 3 S/W-Kameras im EDL und eine Farbkamera in KAL. Man beachte, dass dann größere Informationsmengen verschoben werden müssen, was die Erfassung langsamer macht. Das gleiche Argument spricht auch gegen höher auflösende Kameras, die grundsätzlich geringere Bildraten bieten. Bei der in den Varianten 4 und 4b dargestellten Ausführungsform (siehe Figuren 16 und 23) ist der Einsatz höher auflösender Kameras bevorzugt, da hier nur ein Teil des Bildes genutzt werden kann und sowieso nicht alle den Strömungsquerschnitt durchfließenden Partikeln erfasst werden können. Eine eineindeutige Zählung (jede Partikel genau einmal) ist nicht für alle Anwendungen zwingend, jedoch dann wünschenswert, wenn das erfasste Volumen mit dem Gewicht der Probe zur Ermittlung der Scheindichte genutzt werden soll (siehe auch Punkt bae)). Dazu müssen die Länge der Förderstrecke (Bildausschnitt), Fördergeschwindigkeit und Bildrate abgestimmt werden, damit jede Partikel mindestens einmal vollständig gesehen wird. Es können beispielsweise 25 Bilder/sec realisiert werden. Für die zeitrichtige Erfassung der Bilder (siehe bae) und bbb)) sind grundsätzlich alle Kameras geeignet, die sich synchron betreiben lassen; ansonsten müsste man die Partikelbewegung im richtigen Moment anhalten, was nicht immer praktikabel und auf jeden Fall langsam ist.

*bae) Zeitliche Eingrenzung*

Da die Partikel kontinuierlich gefördert werden, sind, um besonders gute Ergebnisse zu erhalten, zwei Randbedingungen bevorzugt:
Zum einen sollte die Belichtungszeit so kurz sein, dass keine Bewegungsunschärfe entsteht. Bei mittleren Abbildungsmaßstäben und einer lichtstarken EDL Anordnung ist dies bereits mit Dauerlicht und Shutterbetrieb der Kamera möglich. Bei höheren Auflösungen kann alternativ ein Blitz eingesetzt werden. Dabei ist es möglich, den Blitz auf mehrere Beleuchtungen aufzuteilen.

Bezüglich des Zeitpunkts und der Häufigkeit der Bilder gelten die folgenden Aspekte zur Erzielung optimaler Ergebnisse bei Anwendung des erfindungsgemäßen Verfahrens. Selbst wenn man die Dosierung der Partikel aktiv steuert (einzeln, "particle on demand"), kann man den Zeitpunkt, an dem sich die Partikel in Bildmitte befinden wird, nicht genau vorsehen. Daher ist es schwierig, mit Anordnungen, die nicht in einer Ebene liegen und deshalb nur einen Schärfepunkt besitzen, den Zeitpunkt zu treffen, an dem die Partikel gerade für alle Beobachtungen scharf ist. Die Möglichkeit, aus einer Hochgeschwindigkeits-Aufnahme (1 pro Richtung!) die guten Bilder auszuwählen, ist möglich, jedoch wegen dem damit verbundenen Datenfluss nicht bevorzugt. Grundsätzlich kann man mit einer Lichtschranke kurz vor dem Messort triggern.

Wesentlich einfacher und daher besonders bevorzugt ist die Realisierung, wenn alle Beobachtungen in einer Ebene liegen. Dann haben alle Bilder eine gemeinsame Längsachse und es ist unbedeutend, wo genau sich die Partikel zum Zeitpunkt der Abbildung befindet. Es sollte bevorzugt nur sichergestellt werden, dass die Aufnahmen häufig genug erfolgen, damit jede Partikel mindestens einmal vollständig erfasst wird, falls eine eineindeutige Zählung benötigt wird (d.h. für jede Dosierung wird ein Bildersatz aus mindestens einem Bild pro Beobachtungsrichtung angelegt). Durch Unvollkommenheit der Dosierung, sowie Bruch oder Desagglomeration können aber auch zwei oder mehr Partikeln gleichzeitig oder leicht zeitversetzt durchlaufen.

*bb) Bilderfassung*

*bba) Abbildungsmaßstab*

Der Abbildungsmaßstab ergibt sich aus der Chipgröße der Kamera, wobei bevorzugt eine CCD oder CMOS-Kamera eingesetzt wird, die im Allgemeinen zwischen 0,25 und 1 Zoll liegt, der Pixelzahl der Kamera (0,3 bis 4 Megapixel sind derzeit üblich), und dem Vergrößerungsfaktor der Optik. Dieser ist nach unten unbegrenzt, im Bereich 0,1 bis 1 redet man von Makroobjektiven und oberhalb von 1 bis etwa 100 von Mikro(skop)objektiven. Höher auflösende Objektive haben in der Regel so geringe Arbeitsabstände, dass es technisch nicht möglich ist, den gleichen Punkt simultan aus verschiedenen Richtungen abzubilden, da sich die Objektive dann gegenseitig im Weg stehen würden (schlanker bauende Sonderausführungen sind physikalisch möglich, aber wirtschaftlich nicht sinnvoll). Anders als bei einer reinen Größenmessung, wo man bereits mit sehr geringen Pixelzahlen (als Länge einer Partikel betrachtet) von 2-10 zufrieden sein kann, benötigt man für die Formbeschreibung deutlich mehr Auflösung. Dies hängt von der Komplexität der jeweiligen Form ab, aber Zielwerte in der Größenordnung von im Allgemeinen 10 bis 200 Pixel, bevorzugt 50 bis 200 Pixel sind angemessen.

*bbb) Bildauswahl für Verarbeitung und/oder Speicherung*

Es ist vorteilhaft, nur solche Bilder zu verarbeiten und/oder zu speichern, die überhaupt Partikeln zeigen, und leere Bilder zu verwerfen. Es genügt, eines der Kamerabilder abzufragen, ob sich darin wenigstens eine Partikel aufhält. Nur dann wird abgespeichert und/oder ausgewertet. Da auch Partikeln, die den Rand berühren, verworfen werden müssen, kann man dies gleichzeitig abfragen.

Für das weitere Vorgehen gibt es mehrere Strategien. Bevorzugt werden alle Bildersätze zunächst schnell in den Arbeitsspeicher abgelegt, und wenn nach einer angemessenen Wartezeit (0.1 bis 3 sec, je nach Produkt) keine Nachzügler mehr zu erwarten sind, wird ein Bildersatz für das Auswerten und/oder Abspeichern auf Datenträger ausgewählt.

Die Freigabe für die nächste Dosierung erfolgt bevorzugt erst dann, wenn alle vorangehenden Aufgaben abgeschossen sind. Es ist bei großer Eile aber möglich, den Abruf schon etwas früher zu starten, wenn das Ende der Bearbeitung absehbar ist. Im allgemeinen Fall wird man mehrfache Abbildungen oder Koinzidenzen (2 oder mehr Partikeln gleichzeitig) in einer zeitunkritischen Nachbearbeitung bearbeiten. Da für eine Doppelerkennung die 3D Auswertung die besten Kriterien liefert, ist die Auswertung nach jeder Dosierung bevorzugt. Es ist dann möglich, aus mehreren Bildersätzen nach geeigneten Kriterien auszuwählen, zum Beispiel den Bildersatz mit dem geringsten Volumen der Partikel, oder mit der größten Anzahl verschiedener Partikeln. (d.h. den Satz, wo zwei koinzidente Partikeln als getrennt erkennbar sind.)

Je nach eingesetzter Hardware kann es aber auch wünschenswert sein, alle oder nur grob vorausgewählte Bilder zu speichern.

Bei einer Speicherung ist es stets das Ziel, die Datenrate zu maximieren, was einen Kompromiss zwischen komprimierender Vorverarbeitung und Schreibgeschwindigkeit auf die Platte erfordert. Die Methoden dazu sind dem Fachmann bekannt. Grundsätzlich kann man die Datenströme möglichst früh vereinfachen, und sie auf verschiedene Framegrabber, Schnittstellen, Prozessoren und Festplatten verteilen. Vorteilhaft ist auch eine doppelt gepufferte Bildtriggerung, Bildbelichtung, Bildübertragung, Bildverarbeitung und Bildspeicherung. Damit sind im Allgemeinen Bildraten in der Größenordnung von 40 ms möglich, bei einfachen Systemen langsamer, bei Einsatz von leistungsfähiger Hardware auch deutlich schneller (z.B. 5-10 ms). Diese Bilder können in verschiedenen, zum Teil komprimierenden Aufzeichnungsverfahren (Fileformaten wie TIFF, JPEG, AVI) aufgezeichnet werden. Bei gut bekannten Materialien kann man z.B. schon im Kameratreiber oder auf dem Framegrabber das jeweilige Bild mit einer Schwelle binärisieren, so dass bis zu 8 einzelne Binärbilder in die 8 Bits eines üblichen 8-Bit-Bildes mit 256 Graustufen gelegt werden können. In diesem Fall muss in der nachfolgenden Bearbeitung nicht mehr eine Grau-Schwelle gebildet werden, sondern die einzelnen Ebenen müssen extrahiert werden, was aber ebenfalls mit einer Schwellenoperation erfolgt. Ein gegebenenfalls in Auflicht gewonnenes S/W- oder Farbbild muss als Grau- oder Farbbild gespeichert werden, um später ausgewertet werden zu können.

*c) Auswertung der Bilder*

*ca) Vorverarbeitung der Bilddaten*

Gegebenenfalls, je nachdem, welche Voraussetzungen bei der Erfassung gegeben waren, werden die gespeicherten - bzw. bei direkter Verarbeitung (Auswertung) der im Anschluss an die Bildvorauswahl erhaltene Bildersätze bevorzugt noch vorverarbeitet. Folgende Operationen sind sowohl vorher als auch nachträglich realisierbar:

- Schwellenbildung (Binärbild aus Grau- oder Farbbild oder aus Bitebene s.o.)

- Bildreinigung (Erosion, Dilatation, Verwerfen von Staubpartikeln im Bild)

- Löcher Füllen (z.B. bei transparenten Partikeln)

- Randgängige Partikeln verwerfen

- Doppelt gezählte Partikeln verwerfen
  Mehrfache Abbildungen einer Partikel können durch Ähnlichkeitsbetrachtungen eliminiert werden. Als Kriterium können alle erfassten Partikeleigenschaften herangezogen werden und Zeit und Ort der Erfassung. Da sich die Partikeln stets vorwärts bewegen, muss z.B. eine Partikel, die "hangaufwärts" auftaucht, eine neue sein. Die Toleranzen für die Entscheidung, ob eine Partikel "neu" ist, müssen an die Unterschiede zwischen den Partikeln und die Wahrscheinlichkeit, dass eine Dosierung mal mehr als eine Partikel liefert, angepasst werden (Die besten Kriterien liefern die orientierungsunabhängigen 3D Parameter, man beachte die Ausführungen unter bbb))

- Aus Auflichtbild Kontur (Binärbild) und Farbinformation extrahieren Das Binärbild muss mit einer Schwellenbildung erkannt werden (Kontur für Rekonstruktion) und gleichzeitig muss die Farbe innerhalb dieser Kontur bestimmt werden (Mittelwerte, Standardabweichungen und Verteilungen von Graustufen, oder Farben, z.B. im RGB oder HSL-System (oder in anderen dem Fachmann bekannten Farbräumen).

- Partikeln mit ungünstiger Lage verwerfen (siehe auch Auswahlkriterien unter bbb)

- Partikelbilder individuell auf eine sinnvolle Auflösung reduzieren

- Bilder von Koinzidenzen auseinanderdividieren (s.o.)
  In der Regel legt man die Probenförderung so aus, dass nur jeweils eine Partikel abgegeben wird und Doppelereignisse (oder Mehrfachereignisse, auch Koinzidenzen genannt) selten sind. Viele Doppelereignisse werden dadurch erkannt, dass sich zwei Partikeln auch mal gleichzeitig im Bildfeld befinden, oder sich signifikant unterscheiden.

*cb) Rekonstruktion des Volumens:*

Zur Rekonstruktion und Darstellung der individuellen dreidimensionalen Form der Partikeln (3D Körper) aus den in Schritt b) erhaltenen und gegebenenfalls in Schritt ca) bearbeiteten Projektionsbildern können alle dem Fachmann bekannten Verfahren eingesetzt werden, zum Beispiel Weichert, R., Huller, D.: Volumenbestimmung und Formerkennung unregelmäßig geformter Partikeln mittels dreidimensionaler Bildanalyse; Nürnberg, 2. Europ. Symposium Partikelmesstechnik (1979), Seite 266 - 272. Es sei erwähnt, dass die jeweils sichtbaren Projektionsflächen einen räumlichen Körper begrenzen; und man könnte, jeweils einer Beobachtungsrichtung folgend, alle die Bildbereiche "wegfräsen", worin kein Partikelvolumen sichtbar ist. Dies ist zwar korrekt, aber mathematisch komplex uns sehr zeitaufwendig. Sehr effizient und bevorzugt ist dagegen eine erfindungsgemäße Rekonstruktion nach dem Konzept der "Scheiben", die nachstehend näher erläutert ist.

Die Rekonstruktion des Volumens ist besonders genau für vollständig konvexe Körper. Körper mit konkaven Anteilen können durch Projektionsflächen nur eingeschränkt erfasst werden, wobei man allerdings im räumlichen Bereich zwischen einachsigen und zweiachsigen konkaven Anteilen unterscheiden muss. Zweiachsig konkav ist ein "Loch", seine Tiefe kann aus keiner seitlichen Richtung erfasst werden. Einachsige konkave Anteile, d.h. "Gräben" und Einschnürungen, können aus der geeigneten Perspektive erfasst werden. Als Beispiel kann man hier eine Doppelkugel anführen, deren Einschnürung in lateraler Ansicht (Kugeln nebeneinander) vollständig einsehbar ist, während sie in Frontansicht verborgen bleibt.

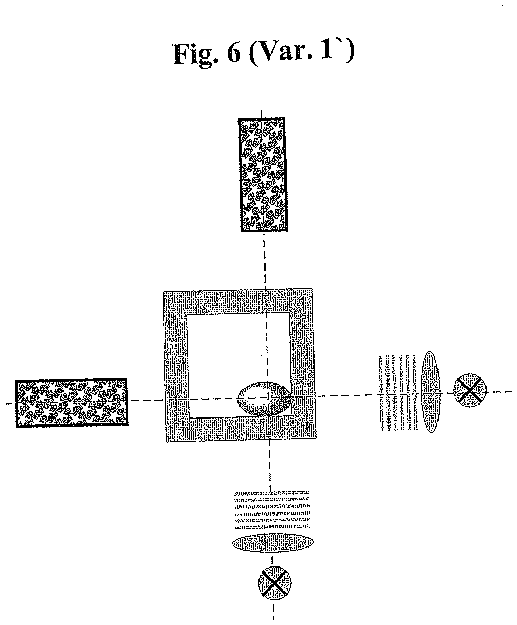

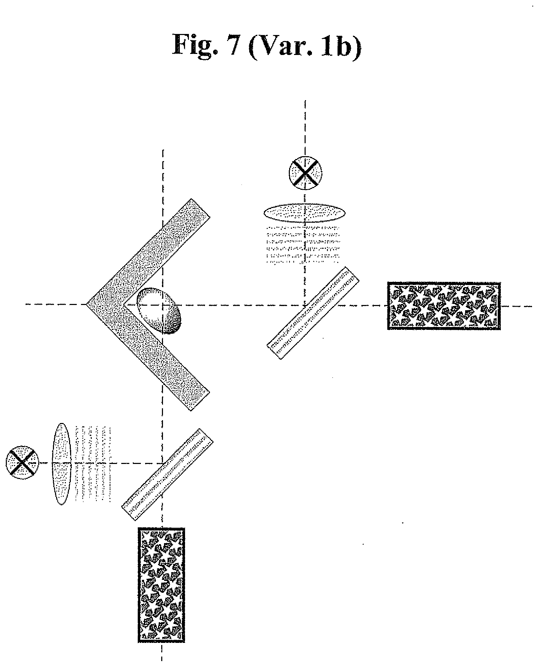

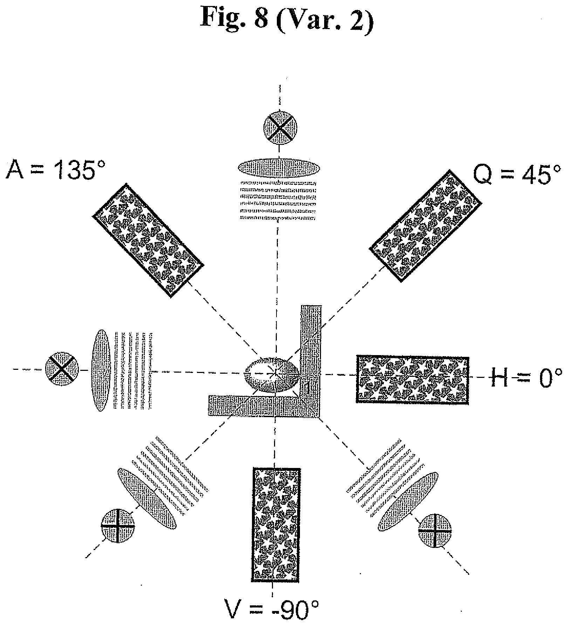

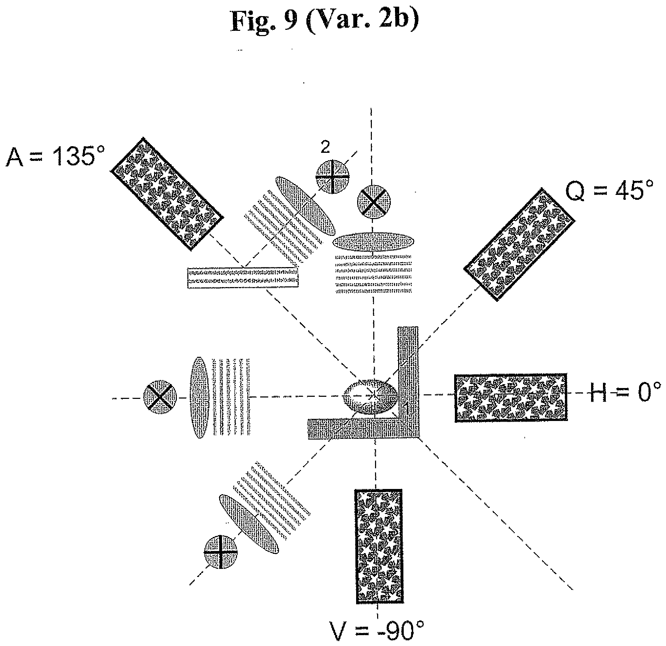

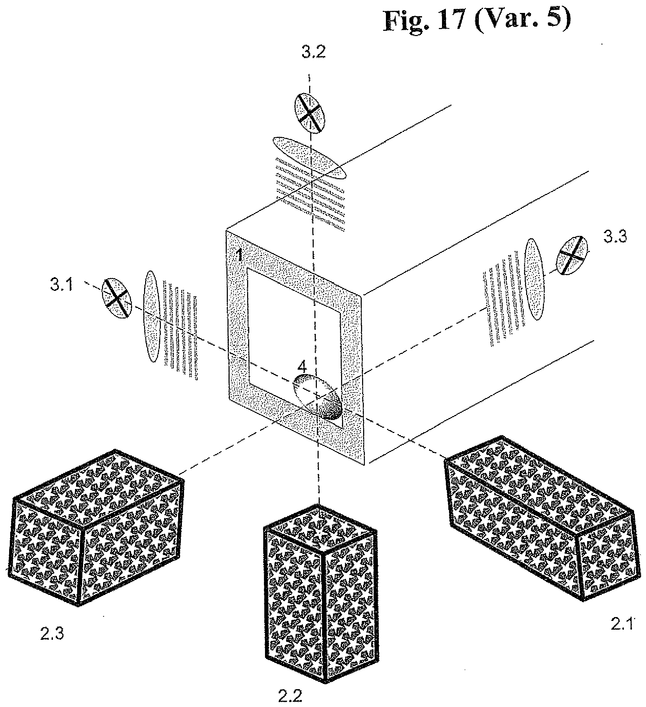

Alle Beobachtungen, die in einer Ebene quer zur Ausrichtung (und Bewegung) erfolgen (Variante 1, Figur 5; Variante 1', Figur 6; Variante 1b, Figur 7; Variante 2, Figur 8; Variante 2b, Figur 9; Variante 2c, Figur 10; Variante 4, Figur 16; Variante 4b, Figur 23), haben eine gemeinsame Längsachse, hier sei sie Z genannt. Alle Bilder setzen sich aus Sehnen senkrecht zu dieser gemeinsamen Achse zusammen, alle Sehnen über einer Z-Koordinate beschreiben eine Scheibe des Körpers (ein Voxel dick). Durch die Ausrichtung in Längsrichtung erfassen die Beobachtungen also eine Reihe von Scheiben für das Gesamtvolumen. Auch bei vielen an sich konkaven Formen sind diese einzelnen Scheiben nicht konkav, nur unterschiedlich groß, geformt und positioniert. Eine zusätzliche Beobachtung von vorne (siehe z.B. Variante 5, Figur 17) ist nur dann von Vorteil, wenn der Querschnitt konstant ist (was in der Regel nur bei Profilstränglingen der Fall sein wird) und konkave Körperdetails nur in Längsrichtung einsehbar sind.

Mit diesem Konzept kann man den Nutzen der Anzahl N Beobachtungsrichtungen genau bestimmen. Zur Erläuterung seien hier symmetrische Querschnitte angenommen, und gleichmäßig verteilte Winkel und zwar 360°/2*N, d.h. z.B. 90° für N=2, usw.:
Zwei Sehnen der Länge L begrenzen bei 90° ein Quadrat der Seitenlänge L als maximalen, sowie ein 45° gedrehtes Quadrat (also halber Fläche) und Diagonale L als minimale konvexe Fläche. Die Unsicherheit beträgt also einen Faktor 2.

Verallgemeinert kann man sagen, N Beobachtungsrichtungen ergeben ein Polygon mit 4*N Ecken, bei 2*N dieser Ecken haben die Sehnen zwischen gegenüberliegenden Ecken die Länge L, und bei den anderen liegt diese Länge zwischen $L*\cos\alpha$ □und $L/\cos\alpha$, wenn $\alpha$ als 360°/4*N definiert wird. Das Fehlerverhältnis E ergibt sich somit zu $\cos^2(\alpha)$,
d.h
für N=2 ist E=2.00 (s.o.)

für N=3 ist E=1.33

für N=4 ist E=1.17

Ohne Vorkenntnis über das Produkt ist demnach L oder der Mittelwert von Max- und Min-wert die günstigste Schätzung. Bei Produkten mit bestimmten, durch planparallele Flächen bestimmten Formen kann aber auch L-Max der günstigere Wert sein. Bevorzugt wird daher der Bereich zwischen L-Min und L-Max durch einen Anpassungsparameter von 0-100% angewählt, der im Normalfall bei 50% steht. Diese Überlegungen können durch einfache Anwendung ebener Geometrie auf die Situation nicht gleichverteilter Winkel (deren Einsatz aber nichtvorteilhaft ist) und nichtsymmetrischer Querschnitte (was der allgemeine Fall ist) übertragen werden.

Ein weiterer Gegenstand der vorliegenden Erfindung ist ein Verfahren zur Rekonstruktion des Volumens von individuellen Partikeln bei einer Beobachtung der Partikeln aus N Beobachtungsrichtungen in einer Ebene quer zur Ausrichtung der Partikeln, wobei das Volumen der individuellen Partikel aus ein Voxel dicken einzelnen Scheiben in Form eines Polygons mit 4*N Ecken zusammengesetzt wird und der Querschnitt der einzelnen Scheiben mittels der folgenden Schritte ermittelt wird :

a) Ermittlung des maximal möglichen Querschnitts Q-MAX, bevorzugt durch Verbinden der 2*N Schnittpunkte der jeweils zwei maximalen Begrenzungslinien in den N Beobachtungsrichtungen, wobei ein Polygon MAX mit 2*N Ecken und die 2*N Ecken verbindenden Seiten L-MAX erhalten wird;

b) Ermittlung des minimal möglichen Querschnitts Q-MIN, bevorzugt durch Verbinden der Seitenmittelpunkte des in Schritt a) erhaltenen Polygons mit 2*N Ecken, das den maximal möglichen Querschnitt bildet, wobei ein Polygon MIN mit 2*N Ecken und die 2*N-Ecken verbindenden Seiten L-MIN erhalten wird;

c) Ermittlung des wahrscheinlichsten Querschnitts Q-OPT einer das Volumen einer individuellen Partikel bildenden Scheibe, der einen Wert von mindestens Q-MIN und maximal Q-MAX aufweist, bevorzugt durch Auswahl eines Querschnitts der durch 4*N Ecken gebildet wird, von denen 2*N die oben genannten MIN-Eckpunkte sind, und die restlichen 2*N auf der Linie zwischen den Mittelpunkten der 2*N-MIN-Seiten und den 2*N-MAX Punkten liegen, bevorzugt auf halber Strecke.

Die Ermittlung der Werte für Q-MIN, Q-MAX, L-MIN und L-MAX erfolgt durch dem Fachmann bekannte Anwendung der Geometrie.

Das erfindungsgemäße Rekonstruktionsverfahren wird bevorzugt in dem erfindungsgemäßen Verfahren zur automatisierten Bestimmung der individuellen dreidimensionalen Form von Partikeln in Schritt c) eingesetzt.

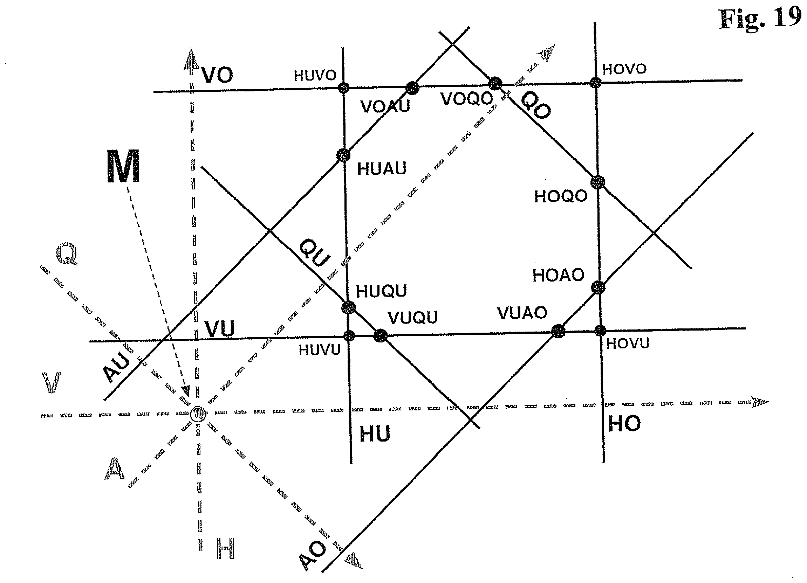

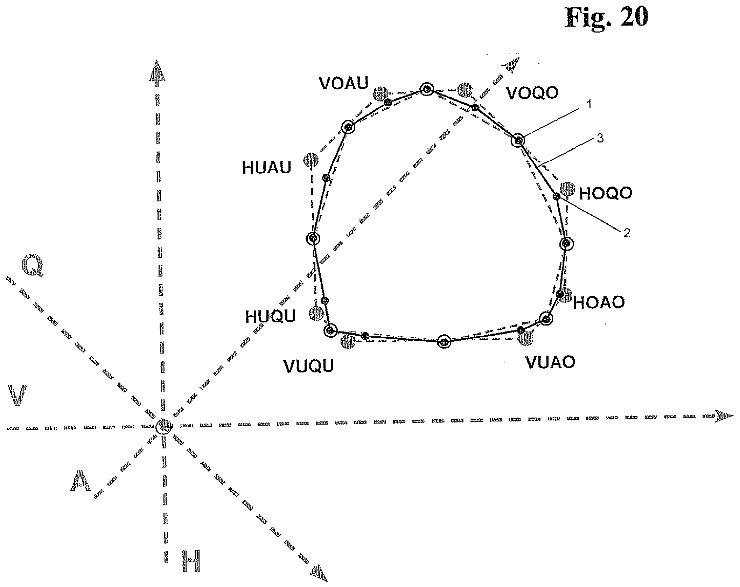

Für die bevorzugte Ausführung mit 4 Beobachtungsrichtungen ergibt sich ein 16-Eck. Dieses wird konstruiert aus den 8 Schnittpunkten HUAU, VOAU, VOQO, HOQO, HO-AO, VUAO, VUQU und HUQU zwischen den Beobachtungsstrahlen (siehe z.B. Figuren 19, 20). Dieses 8-Eck ist der maximal mögliche Querschnitt. Die 8 Seitenmittelpunkte 1 dieses 8-Ecks bilden das minimal mögliche 8-Eck (siehe Figur 20). Setzt man nun einen beliebigen Punkt auf der Linie von den 8-Seitenmittelpunkten des aus den Seitenmittelpunkten 1 gebildeten 8-Ecks zu den zugehörigen Eckpunkten HUAU, VO-AU, VOQO, HOQO, HOAO, VUAO, VUQU und HUQU zusätzlich als Stützpunkte 2 ein, wobei sie auf diesen Strecken mit einem Parameter von 0 bis 100% definiert verschoben werden, so kann man den Querschnitt stufenlos zwischen minimalem und maximalem Querschnitt einstellen. Im Allgemeinen ist ein Wert von 50% vorteilhaft.

Für die einfachere Ausführungsform mit 2 Beobachtungsrichtungen gibt es eine weitere bevorzugte Rekonstruktion: es wird ein so genannter Rekonstruktionsquerschnitt ausgewählt. Dazu werden zwei Rekonstruktionsquerschnitte oval (E) oder rechteckig (R) in Kenntnis der zu messenden Partikel vorgegeben. Für bestimmte bekannte Volumenformen prüft das Programm daran die Querschnitte der jeweiligen Bilderpaare und ordnet ihnen entweder einen Querschnitt R oder einen Querschnitt E zu, indem für jedes Partikelbild ein zweidimensionaler Formfaktor ermittelt wird. Dies ergibt die Kombinationen EE, RR und RE bzw. ER. Der dritte Querschnitt wird automatisch nach dem vorgegebenen Körpermodell ergänzt, z. B. RRR (Quader), EEE (dreiachsiges Ovaloid), RRE (Zylinder) oder REE (kissenförmiges Partikel).

Ein weiterer Gegenstand der vorliegenden Erfindung ist daher ein Verfahren zur Rekonstruktion des Volumens von individuellen Partikeln bei einer Beobachtung der Partikeln aus zwei Beobachtungsrichtungen im Allgemeinen mit Hilfe von zwei Kameras, das die folgenden Schritte umfasst:

i) Auswahl eines Rekonstruktionsquerschnitts durch Vorgabe von zwei Rekonstruktionsquerschnitten, oval oder rechteckig, in Kenntnis der zu bestimmenden Partikeln;

ii) Automatisierte Prüfung der Querschnitte der in Schritt b) erhaltenen jeweiligen Bilderpaare anhand der vorgegebenen Rekonstruktionsquerschnitte und automatisierte Zuordnung eines der vorgegebenen Querschnitte oval oder rechteckig;

iii) Automatisierte Ergänzung des dritten Querschnitts gemäß den Vorgaben unter i), wobei für jede individuelle Partikel ein 2-dimensionaler Formfaktor ermittelt wird.

Das erfindungsgemäße Rekonstruktionsverfahren wird bevorzugt in dem erfindungsgemäßen Verfahren zur automatisierten Bestimmung der individuellen dreidimensionalen Form von Partikeln in Schritt c) eingesetzt.

Im Anschluss an Schritt cb) liegt jeweils eine Abbildung der individuellen Partikel unter Rekonstruktion eines dreidimensionalen Bildes jedes Partikel vor. Bevorzugt handelt es sich bei dem dreidimensionalen Bild um ein Voxelbild.

*cc) Weitere Auswertungen*

Die Ermittlung des tatsächlichen Volumens der jeweiligen Teilchen ist schwierig, da man Vertiefungen (Löcher, Gräben, Einschnürungen) sowie Toträume hinter Nasen in der Oberfläche der Partikel nur schwer erkennen kann, diese aber einen wesentlichen Einfluss auf das tatsächliche Volumen der Teilchen haben. Somit umfasst das Softwareprogramm zur Ermittlung des tatsächlichen Volumens jedes Teilchens Anpassungsparameter, die je nach der bekannten Form der Teilchen angenommen werden, und entsprechende Extrapolationen vorzunehmen (Konvexkorrektur). Aus den konkaven Anteilen der Projektionsflächen kann man die nicht erfassten Volumenanteile schätzen. Verschiedene Modellüberlegungen mit "Brombeeren" und Würfeln mit aufliegenden kleinen Würfeln zeigen, dass ein lineares Modell gut geeignet ist:

$$\text{Konkaver Anteil im Volumen} = \text{Parameter} * \text{konkaver Anteil in der Projektionsfläche},$$

mit einem Anpassungsparameter bevorzugt zwischen 0,2 und 4. Diese Werte wurden durch geometrische Modellrechnungen ermittelt.

Für Simulationsrechnungen ist die Darstellung des Partikelvolumens durch einen Satz von sich durchdringenden Kugeln vorteilhaft. Diese Kugeln werden automatisch ermittelt. Folgende Vorgehensweise ist bevorzugt: für jede Voxel des Partikelvolumens wird der Abstand zur Partikeloberfläche $R_0$ bestimmt. Von dem Punkt mit dem höchsten Wert wird in einen separaten Bildspeicher eine Kugel mit einem Radius gezeichnet, der bevorzugt geringfügig größer ist (R_Draw), und Mittelpunktskoordinaten sowie Radius werden gespeichert. Aus dem Originalbild wird diese Kugel gelöscht (bzw. vorteilhaft eine etwas kleinere Kugel mit dem Radius R_Delete). Für das verbleibende Originalbild wird wieder das Voxel mit dem höchsten Wert gesucht, und die Prozedur wiederholt sich, bis entweder die vorgegebene Anzahl Kugeln erreicht ist, oder ein bestimmter Prozentsatz des Originalvolumens im neuen Bild erreicht ist (z.B. 98%), oder der kleinste zulässige Radius, oder im Originalbild alle Voxel abgebaut sind. Diese Parameter wurden für bisher untersuchte Produkte experimentell ermittelt (Vergleich: gemessenes Volumen vs. von den Kugeln wiedergegebenes Volumen).

Folgende Werte haben sich z.B für ein rauhes Produkt, mit 0.3 bis 3 mm Partikelgrößen bewährt (mit 400 Kugeln erfasst):

$$\text{R\_Draw} = R_0 * \text{CDraw} + \text{Offset}$$

wobei C-Draw Werte zwischen 100% und 102 % annimmt, und Offset Werte zwischen 0.60 und 0,75 Pixel

$$\text{R\_DELETE} = R_0 * \text{CDelete}$$

wobei C-Delete Werte zwischen 80% und 85% annimmt.

Für andere Produkte und Auflösungen sind die Werte nach der oben beschriebenen Vorgehensweise zu ermitteln.

Die Bestimmung der längsten Sehnen und anderer geometrischer Parameter ist in der Literatur beschrieben und dem Fachmann bekannt.

Eine besondere Lösung wurde erfindungsgemäß für die Bestimmung der Oberfläche entwickelt. In 2D Bildern wird der Umfang vorteilhaft ermittelt mit einer Wertetabelle, die jeder Pixelkombination einen festen Wert zuweist (siehe Michael Schäfer; Digital Optics: Some Remarks on the Accuracy of Particle Image Analysis (p 158-168), Part.Part.Syst.Charact. Vol 19, Issue 3, July 2002). In 2D hat ein Pixel 8 Nachbarn, so dass 256 möglichen Mustern ein Wert zuzuweisen ist.

Dieses Prinzip in 3D zu überführen ist nicht praktikabel, da die 26 räumlichen Nachbarn in einer 3x3x3 Nachbarschaft 67108864 Kombinationen einnehmen können, die Liste wäre kaum zu erstellen und zu handhaben. Daher wurde erfindungsgemäß eine Oberflächenzuordnung auf Basis einer 2x2x2 Nachbarschaft gefunden. Zunächst wird ermittelt, welche der 256 Kombinationen äquivalent sind, hierbei ist zu beachten, dass sie sich durch Rotation und Spiegelung ineinander umwandeln lassen, so dass es tatsächlich nur 22 verschiedene Muster gibt. Nur 3 dieser Grundformen kommen bei in X, Y, Z ausgerichteten Quadern vor: glatte Flächen, gerade Kanten, Ecken. Diese Werte werden dann für verschiedene Seitenverhältnisse und Größen so gesetzt, dass sich exakt die geometrisch richtige Oberfläche ergibt. Somit ergibt sich dann für diese Ecken der Wert 0.75, für diese Kanten der Wert 1, und für diese Flächen ebenfalls der Wert 1. Dann werden die restlichen Werte so gesetzt, dass eine möglichst gute Erfassung von Kugeln oder anderen vorausgesetzten Formen resultiert. Bei Kugeln beträgt der optimale Wert für Ecken z.B. 0.6, für Kanten 0.8 und für Flächen 0.965. Nach dieser Vorschrift lassen sich geeignete Werte für alle Muster ermitteln. Für jede Formfamilie werden Testkörper in verschiedener Parametrisierung (Durchmesser, Abmessungen), Lage und Orientierung, aber mit bekannter Oberfläche erzeugt und mit den Startwerten der Parameter ausgewertet. Dann werden die Parameter variiert, bis der Gesamtfehler minimiert ist. Dies kann manuell/interaktiv/intuitiv erfolgen, oder nach bekannten mathematischen Verfahren der Fehlerminimierung.

Ein weiterer Gegenstand der vorliegenden Erfindung ist somit ein Verfahren zur Bestimmung der Oberfläche von individuellen Partikeln anhand von 3D Voxelbildern auf Basis einer 2x2x2 Nachbarschaft, das die folgenden Schritte umfasst:

i) Ermittlung von äquivalenten Kombinationen aus der Gesamtzahl von 256 möglichen Kombinationen, wobei 22 verschiedene Muster ermittelt werden;

die weitere Optimierung der Werte für die 22 Muster kann entweder nach dem Fachmann bekannten Standardverfahren erfolgen oder nach dem nachstehend beschriebenen Verfahren umfassend die Schritte ii), iii) und iv):

ii) Ermittlung der Häufigkeit der einzelnen Muster für einen bestimmten Satz von Testkörpern;

iii) Optimierung der Werte für die einzelnen Muster in der Reihenfolge ihrer Häufigkeit ausgehend von einem Wertebereich von 0 bis 2, bevorzugt 0 bis 1;

iv) Iteration bis zu konstanten Werten mit einem Restfehler von im Allgemeinen im Mittel maximal 2%, bevorzugt im Mittel maximal 1%, so dass sich eine optimale Erfassung der Oberfläche der individuellen Partikel ergibt.

Unter einem bestimmten Satz von Testkörpern (siehe ii)) ist die Form der zu vermessenden Partikeln zu verstehen.

Der Wert des Restfehlers (siehe iv)) ist unter anderem Abhängig von den jeweiligen Testkörpern. Für Kugeln wird im Allgemeinen ein Restfehler von im Mittel maximal 1% erzielt.

Geeignete Optimierungsverfahren (siehe iii)) und Iterierationsverfahren (siehe iv)) sind dem Fachmann bekannt.

Neben diskreten Volumenelementen wie kubischen Voxeln kann die Form der individuellen Partikeln auch durch finite Flächenelemente wie Dreiecke dargestellt werden, wie vorstehend bereits erwähnt wurde. Bei einer Darstellung durch finite Flächenelemente erfolgt die Bestimmung der Oberfläche der individuellen Partikeln mit Hilfe von dem Fachmann bekannten Algorithmen, wobei im Allgemeinen eine noch höhere Genauigkeit erzielt werden kann als in den Verfahren zur Bestimmung der Oberfläche der individuellen Partikeln ausgehend von 3D Voxelbildern.

Die Ergebnisse der Auswertung in Schritt c) können z. B. für die folgenden Fragestellungen genutzt werden:

i) Beurteilung von Produkten anhand verschiedener Eigenschaften (Verteilungen, Mittelwerte)

ii) Beurteilung von Produkten und Unterschieden anhand von Eigenschaftsprofilen (Fingerprint)

iii) Bestimmung der Korrelation verschiedener Produkt- bzw. Partikeleigenschaften

iv) Bestimmung von Produkteigenschaften und Produktverhalten durch numerische Simulationen, z. B. Schüttdichte, Lösekinetik, Verbackungsverhalten, Rieselverhalten, Siebfraktionen

*Bevorzugte Ausführungsformen der vorliegenden Erfindung*

Im Folgenden sind besonders bevorzugte Ausführungsformen der vorliegenden Erfindung aufgeführt. Es sind jedoch zahlreiche weitere Ausführungsformen für den Fachmann aufgrund des vorstehend genannten erfindungsgemäßen Verfahrens und der erfindungsgemäßen Vorrichtung leicht zu ermitteln. Es ist daher nicht beabsichtigt, die Erfindung auf die nachstehend genannten Ausführungsformen zu begrenzen.

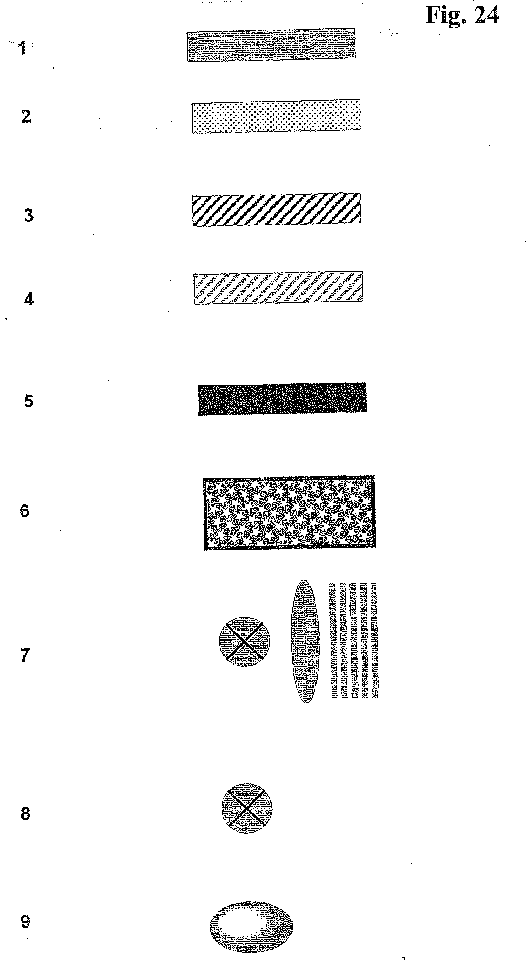

In Figur 24 sind die in den nachfolgend dargestellten Varianten (Ausführungsformen) eingesetzten Materialien zusammengefasst.

In den Figuren 5 bis 18 und 23 sind bevorzugte Ausführungsformen der vorliegenden Erfindung beispielhaft dargestellt:

*Figuren 5 bis 7*

a. Einfache orthogonale Anordnung mit 2 Kameras in EDL, mit quadratischer Küvette und Rutschförderung (Varianten 1 (Figur 5) und 1' (Figur 6)), sowie als Auflichtvariante (Variante 1b (Figur 7)). Diese Variante hat den Vorteil, dass die sehr kostengünstig ist.

*Figuren 8 bis 10*

b. 0°/45°/-90°/135° Anordnung mit 4 Kameras in einer Ebene in EDL (Variante 2 (Figur 8)), alternativ mit 0°/90°/45° in EDL und 135° KAL (Variante 2b (Figur 9)), jeweils in rechtwinkliger Glas-V-Rinne und Rutschförderung. Alle in Variante 3 (Figuren 11 bis 15) dargestellten Ringprofile und Anordnungen eignen sich natürlich auch als transparentes oder weiß-diffus streuendes Profil für eine Förderrinne. In Variante 2c (Figur 10) sind für die 135° Richtung beide Beleuchtungswege (KAL und EDL) eingerichtet, sie können wahlweise ohne Umbau genutzt werden, indem die Lampen ein- und ausgeschaltet werden. Man beachte, dass hier die KAL auf 45° geneigte Flächen sieht, so dass kein direkter Reflex entsteht. Diese 2c Variante ist besonders bevorzugt, da sie sehr genau und vielseitig ist, da die 135° Richtung (A) auch mit einer Farbkamera (RGB) ausgestattet werden kann, so dass auch die Farbe der betrachteten Partikel bestimmt werden kann.

*Figuren 11 bis 15*

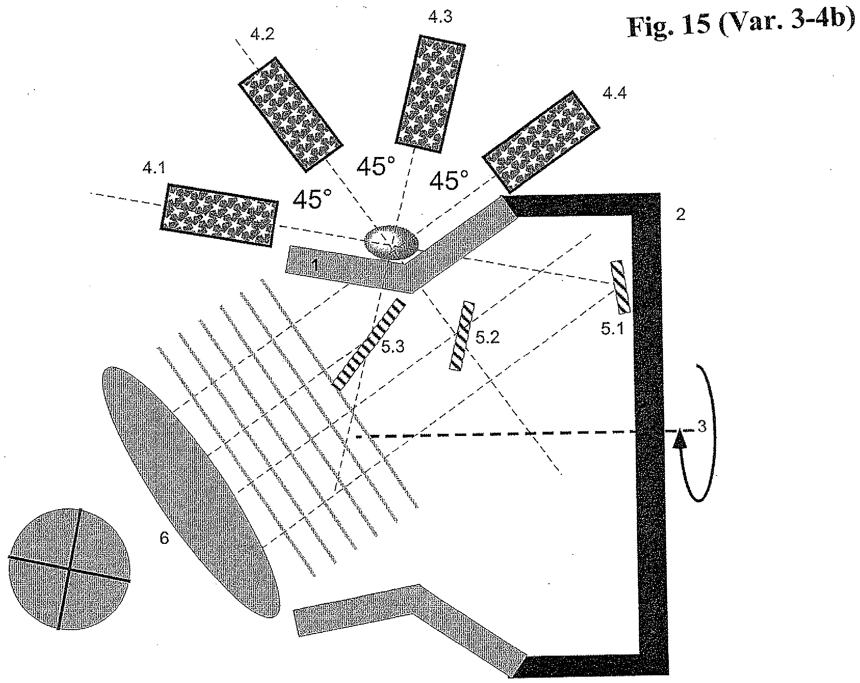

c. Einfache orthogonale Anordnung mit 2 Kameras in DDL, mit rotierendem Zylinder und weiß-diffus streuender, hintergrundbeleuchteter 90°-V-Ring-Nut, Partikelablage außen, Beobachtung auf dem oberen Pol (Variante 3-2 (Figur 11)), alternativ Ablage innen, Beobachtung auf dem unteren Pol (Variante 3-2b (Figur 12)). Wird die Nut mit größerem Öffnungswinkel gewählt, so lassen sich mehr Beobachtungsrichtungen unterbringen, bei 120° 3 im Winkel von jeweils 60° (Variante 3-3 (Figur 13)), sowie bei 135° 4 im Winkel von jeweils 45° (Variante 3-4 (Figur 14), vergleichbar mit Variante 2). Jede dieser Varianten ist auch in EDL realisierbar, dargestellt für 4 Richtungen in Variante 3-4b (Figur 15). Diese Varianten sind bei schwer förderbaren Partikeln bevorzugt, und können wegen der vorgegebenen Fördergeschwindigkeit höhere Partikelraten fahren (mehr Partikeln pro Zeit).

*Figur 16, Figur 23*

d. Einfache orthogonale Anordnung mit 2 Kameras in EDL und einer durchströmten Präzisionsküvette für suspendierte Proben (Variante 4), optische Begrenzung auf Tiefenschärfebereich und vor geschaltete zweiachsige Ausrichtungszelle. Diese Ausführung kann nicht eine eineindeutige Zählung anbieten, sie eignet sich aber gut für kleinere, trocken nicht mehr dosierbare Partikeln und hohe Partikelraten. Alternative Anordnung mit 4 Kameras in einer Ebene (0°/45°/-90°/135°) in EDL, Einsatz einer 8-eckigen Küvette, optisch wie bei Variante 4 oder durch Hüllstromtechnik eingegrenzter Querschnitt (Variante 4b).

*Figuren 17 und 18*

e. Räumlich orthogonale Anordnung (Variante 5 (Figur 17)) mit 3 Beobachtungen in EDL mit jeweils 90° zueinander, eine davon schaut in Förderrichtung (Küvette, Rutsch-/Vibrationsförderung), alternativ (Variante 5b (Figur 18)) auch ausgeführt wie Variante 3-2, aber transparentem Nut-Ring für EDL, und einer tangential angeordneten EDL Beobachtung. Diese Varianten können mit einer Lichtschranke getriggert werden. Diese Varianten sind dann von bevorzugt, wenn die Partikel in Längsrichtung einen konstanten Querschnitt haben.

Ein weiterer Gegenstand der vorliegenden Anmeldung ist eine Vorrichtung zur automatisierten Bestimmung der individuellen dreidimensionalen Form von Partikeln umfassend:

a) Mittel zum vereinzelnden Dosieren der Partikeln, Mittel zum Ausrichten der Partikeln in Längsachse und Mittel zum automatisierten Fördern der Partikeln entlang einer Linie,

b) mindestens zwei Kameras zur Beobachtung der ausgerichteten Partikeln aus mindestens zwei Beobachtungsrichtungen;

c) Mittel zur Auswertung der Bilder.

In den Figuren 21 und 22 sind Beispiele für eine geeignete erfindungsgemäße Vorrichtung in verschiedenen Perspektiven dargestellt.

Geeignete Mittel zum Ausrichten der Partikeln und zum automatisierten Fördern der Partikeln, Mittel zur Bilderfassung und Mittel zur Auswertung der Bilder sind vorstehend bezüglich des erfindungsgemäßen Verfahrens genannt. Anstelle jeweils eines Mittels zum Dosieren, eines Mittels zum Ausrichten und eines Mittels zum Fördern der Partikeln kann auch ein einziges Mittel zum Dosieren, Ausrichten und Fördern eingesetzt werden oder ein Mittel zum Dosieren und ein Mittel zum Ausrichten und gleichzeitigen automatisierten Fördern der Partikeln entlang einer Linie. Geeignete Mittel sind vorstehend genannt.

Geeignete Materialien für die im Strahlengang liegenden Mittel, insbesondere für Förderrinnen und Küvetten, sind vorstehend genannt. Für alle nicht im Strahlengang liegenden Materialien gibt es keine besonderen Anforderungen, jedes Material wie Aluminium, Stahl, Kunststoff, das der Fachmann für feinwerktechnische Konstruktionen einsetzt, ist geeignet. In der Praxis wird meist Aluminium eingesetzt. Auf die verwendeten Materialien wird deshalb nur bei den Förderrinnen/Nut-Materialien eingegangen.

Ein weiterer Gegenstand der vorliegenden Anmeldung ist die Verwendung der erfindungsgemäßen Vorrichtung zur automatisierten Bestimmung der individuellen dreidimensionalen Form von Partikeln. Bevorzugt wird die erfindungsgemäße Vorrichtung zur Durchführung des erfindungsgemäßen Verfahrens verwendet.

**Bezugszeichenliste:**

Fig 1

| | |
|---|---|
| 1.1, 1.2, 1.3 | 3 Beobachtungsrichtungen für Kameras |
| 2 | Beobachtete Partikel |
| 3 | Unterlage, planes Substrat, Träger |

Fig 2a

| | |
|---|---|
| 1.1, 1.2, 1.3 | 3 Beobachtungsrichtungen für Kameras |
| 2.1, 2.2 | Beobachtete Partikeln |
| 3 | Ausschnitt |
| 4 | Bewegungsrichtung (freier Fall) |

Fig 2b

| | |
|---|---|
| 1.1, 1.2, 1.3 | 3 Beobachtungsrichtungen für Kameras |
| 2 | Beobachtete Partikel |
| 3 | Drehteller als Substrat |
| 4 | Bewegungsrichtung Rotation |

Fig 2c

| | |
|---|---|
| 1.1,1.2,1.3 | 3 Beobachtungsrichtungen für Kameras |
| 2 | Beobachtete Partikeln |
| 3 | Scantisch mit Trägerplatte |
| 4 | Bewegungsrichtung X/Y Scan |

Fig 3

| | |
|---|---|
| 1 | Oberste Dosierrinne (optional) |
| 2 | Mittlere Dosierrinne |
| 3 | Untere Dosierrinne |
| 4 | Träger obere Dosierrinne |
| 5 | Träger mittlere Dosierrinne |
| 6 | Träger Gesamtaufbau Dosierrinnen, Verschiebetisch |
| 7 | Grundplatte für Dosierrinnen und Sensormodul |
| 8 | Sensormodul |

Fig 4

| | |
|---|---|
| 1a, 1b | Kommerzielle Dosierrinne, Antriebsteil |
| 2a, 2b | Kommerzielle Dosierrinne, V-Rinne |
| 3 a, 3b | Lichtschrankenhalter mit Lichtschranke |
| 4a, 4b | Trägerplatte für Dosierrinne und Lichtschranke |
| 5, 5a, 5b, 5c | Einbau und Ansichten des Produktführungstrichters |

Fig 5,6,8

1  Küvette als Förderrinne


Fig 9


1  Küvette als Förderrinne
2  Auflichtbeleuchtung für Kamera A = 135°


Fig 10


1  Küvette als Förderrinne
2a  Auflichtbeleuchtung für Kamera A = 135°
2b  Durchlichbeleuchtung für Kamera A


Fig 11


1  weißes, teildurchlässiges Profil
2  Trägerring
3  Rotationsachse


Fig 12


1  weißes, teildurchlässiges Profil
2  Trägerring
3  Rotationsachse
4  Umlenkspiegel


Fig 13


1   weißes, teildurchlässiges Profil
2   Trägerring
3   Rotationsachse
4.1, 4.2, 4.3  3 Kameras im Winkel von jeweils 60°


Fig 14


1   weißes, teildurchlässiges Profil
2   Trägerring
3   Rotationsachse
4.1, 4.2, 4.3, 4.4  4 Kameras im Winkel von jeweils 45°


Fig 15


1   klares, transparentes Profil
2   Trägerring
3   Rotationsachse
4.1, 4.2, 4.3, 4.4  4 Kameras im Winkel von jeweils 45°
5.1, 5.2, 5.3  Umlenkspiegel für die jeweilige Kamera
6   Parallelbeleuchtung mit großer Apertur


Fig 16


1.1  transparente Küvette am Beobachtungsort
1.2  durchströmter Küvettenquerschnitt
2.1  Beleuchtung für Kamera 1
2.2  Beleuchtung für Kamera 2
3.1  Kamera 1
3.2  Kamera 2
4.1  von Kamera 1 berücksichtigter Bildausschnitt

4.2    von Kamera 2 berücksichtigter Bildausschnitt
5      nicht berücksichtigte Partikeln
6      berücksichtigte (gemessene) Partikel
7      Eintrittsquerschnitt der Strömung in den vorgeschalteten Ausrichtungszulauf (zweiachsige Dehnung)

Fig 17

1      transparente Küvette am Beobachtungsort
2.1    Kamera 1
2.2    Kamera 2
2.3    Kamera 3
3.1    Beleuchtung für Kamera 1
3.2    Beleuchtung für Kamera 2
3.3    Beleuchtung für Kamera 3, koaxial in Küvette
4      gemessene Partikel

Fig 18

1           transparentes V-Profil, von innen beleuchtet
2           rotierende Trägerscheibe für V-Profil
3           Rotationsrichtung
4.1, 4.2    Kamera quer zur Drehrichtung, tangential zu den Profilflanken
4.3         Kamera tangential zum Umfang (Nutboden)

Fig 19

M          Koordinatenbezugspunkt für alle Bilder, Küvettenecke
H          Beobachtungsrichtung horizontal, bzw. parallel zu einer Anlagefläche
V          Beobachtungsrichtung vertikal, bzw. parallel zur anderen Anlagefläche
Q          Beobachtungsrichtung quer, d.h. beide Anlageflächen unter 45°
A          Aufsicht, d.h. Blick in die Aussparung bzw. in die V-Rinne
HU, HO     Obere und untere Begrenzung des H-Bildes
VU, VO     siehe oben
QU, QO     siehe oben
AU, AO     siehe oben
HUVU,...   Schnittpunkt zwischen HU und VU, die 4 Punkte HUVU, HUVO, HOVU und HOVO bilden das Rechteck,
           das den maximalen Querschnitt der Bilder H und V umschreibt. Die weiteren Punkte HUAU, VOAU,
           VOQO, HOQO, HOAO, VUAO, VUQU und HUQU ergeben ein Achteck, das den maximalen aus allen
           4 Bildern begrenzten Querschnitt angibt

Fig 20

HUVU,...   HUAU, VOAU, VOQO, HOQO, HOAO, VUAO, VUQU und HUQU ergeben ein Achteck MAX, das den
           maximalen aus allen 4 Bildern begrenzten Querschnitt angibt
1          Die Mittelpunkte der Strecken zwischen jeweils zweien der oberen Punkte (MAX) bilden ein Achteck
           MIN, das den minimalen von 4 Bildern begrenzten Querschnitt angibt.
2          Von den Mittelpunkten der Strecken zwischen jeweils zweien der oberen Punkte (MIN) kann man eine
           Linie zu dem zugehörigen MAX-Punkt ziehen. Auf jeder dieser Strecken kann man einen weiteren
           Stützpunkt des Polygons platzieren. Gezeigt wird der Punkt 2 auf 50% der Strecke vom Minimum zum
           Maximum (HOQO).
3          Das sich ergebende Sechzehn-Eck stellt eine Interpolation zwischen maximal möglichem und minimal
           möglichem Querschnitt dar. Gezeigt ist die Mitte (50%), alle Werte zwischen 0% und 100% sind möglich.
           Ohne Produktkenntnis ist 50% eine gute Annahme, bei mehr Kenntnis über Produkteigenschaften
           können andere Werte bevorzugt werden

Fig 21

1     Trichter von der untersten Dosierrinne zum Einlauf in die Förderrinne (Küvette)

2 Antrieb für die Förderrinne (Lautsprecher mit Schubstange)
3 Höhenjustierung für Antrieb
4 Fuß des Sensors mit Schwenkachse und Winkelskala
5 Höhenjustierung des Küvettenträgers
6 Seitenjustierung des Küvettenträgers (Halterung Federbänder)
7 Schwingende Küvettenhalterung
8 Küvette bzw. V-Rinne
9 Haltering für drehbaren Optik-Block
10 Optik-Block (Fig 22)

Fig 22

1A Kamera der A-Richtung
2A Objektiv der A-Richtung
3A Zentrierfassung für A-Kamera
4A Arretierung Fokusantrieb A
5A Fokusantrieb für A-Kamera
6A Beleuchtungsoptik A-Richtung
7A Kondensor A
8A Streuscheibe/Filter A
9A Kühlblock und Justierfassung für Lichtquelle A
11 Küvette, Förderrinne bzw. V-Rinne in Förderrichtung gesehen, Innenkante stimmt mit optischer Achse überein
12 Trägerkörper mit Aufnahmebohrungen für Kameras und Beleuchtungen

Fig 23

1 Achteckige Küvette
2 Optisch (wie bei Var 4 (Fig. 16)) oder durch Hüllstromtechnik eingegrenzter Querschnitt

Fig 24

1 transparentes, klares Material
2 weißes, diffus streuendes Material
3 Spiegel, 100%
4 Spiegel, halbdurchlässig
5 Undurchsichtiges Material, Metall
6 Kamera, Beobachtung
7 Beleuchtung, parallel
8 Beleuchtung, divergent
9 Partikel

**Patentansprüche**

1. Verfahren zur automatisierten Bestimmung der individuellen dreidimensionalen Form von rieselfähigen Partikeln von Proben in Pulverform, wobei die folgenden Schritte nacheinander durchgeführt werden:

a) Vereinzelnde Dosierung der Partikeln, Ausrichtung der Partikeln in Längsachse und automatisierte Förderung der Partikeln entlang einer Linie;
b) Beobachtung der ausgerichteten Partikeln aus mindestens zwei Beobachtungsrichtungen und Bilderfassung; wobei die Beobachtung mit Hilfe von mindestens zwei Kameras erfolgt;
c) Auswertung der Bilder,

**dadurch gekennzeichnet, dass** die Ausrichtung von Partikeln von Proben in Pulverform gegen zwei plane Anlageflächen, die eine Förderrinne bilden, mit Hilfe der Schwerkraft oder von Zentrifugalkräften erfolgt, und eine Ausrichtung der Partikeln in allen Achsen erfolgt.

2. Verfahren nach Anspruch 1, **dadurch gekennzeichnet, dass** die vereinzelnde Dosierung von Partikeln von Proben

24

in Pulverform mit Hilfe von Dosierrinnen, bevorzugt mit Hilfe von zwei oder mehr hintereinander geschalteten Dosierrinnen, erfolgt.

3. Verfahren nach Anspruch 1, **dadurch gekennzeichnet, dass** als Förderrinne Präzisionsküvetten oder V-Rinnen eingesetzt werden.

4. Verfahren nach einem der Ansprüche 1 bis 3, **dadurch gekennzeichnet, dass** die automatische Förderung von Partikeln von Proben in Pulverform entweder dadurch erfolgt, dass die Partikeln entlang einer Schnittlinie von zwei eine Förderrinne bildenden Flächen entlang einer Linie gleiten, was einen Rutschbetrieb darstellt, oder die Partikeln auf einer bewegten Schnittlinie von zwei eine Förderrinne bildenden Flächen abgesetzt werden, was eine mitlaufende Förderung darstellt.

5. Verfahren zur automatisierten Bestimmung der individuellen dreidimensionalen Form von Partikeln von Proben in Form von Dispersionen, wobei die folgenden Schritte nacheinander durchgeführt werden:

   a) Vereinzelnde Dosierung der Partikeln, Ausrichtung der Partikeln in Längsachse und automatisierte Förderung der Partikeln entlang einer Linie;
   b) Beobachtung der ausgerichteten Partikeln aus mindestens zwei Beobachtungsrichtungen und Bilderfassung; wobei die Beobachtung mit Hilfe von mindestens zwei Kameras erfolgt;
   c) Auswertung der Bilder,

   **dadurch gekennzeichnet, dass** die Ausrichtung und automatisierte Förderung von Partikeln von Proben in Form von Dispersionen in einer Strömungszelle zur zweiachsigen Ausrichtung von Partikeln, umfassend eine Zulaufzone für die Probe enthaltend auszurichtende Partikel und einen Auslass für die Probe enthaltend in zwei Achsen ausgerichtete Partikel, wobei ein Fluidelement der Probe mit den Maßen a, b, c in einer Dehnungszone zu einem Fluidelement mit den Maßen $a \times n$, $b/(n \times m)$, $c \times m$ umgeformt wird, wobei a die Breite, b die Höhe und c die Länge des Fluidelements bedeuten, und n und m von der Geometrie der Strömungszelle abhängige Konstanten, die einem Dehnungsgrad entsprechen, sind, die positive Zahlen > 1 bedeuten, erfolgt.

6. Verfahren nach Anspruch 5, **dadurch gekennzeichnet, dass** die Partikeln von Proben, die in Form von Dispersionen vorliegen, durch einen kleinen Stromfaden, der in eine Strömung durch eine größere Öffnung eingebettet ist, wobei die Strömung durch die größere Öffnung einen Hüllstrom darstellt, geführt werden.

7. Verfahren nach Anspruch 5 oder 6, **dadurch gekennzeichnet, dass** die automatische Förderung der Partikel von Proben, die in Form von Dispersionen vorliegen, mittels einer Pumpe oder eines Druckgefälles erfolgt.

8. Verfahren nach einem der Ansprüche 1 bis 7, **dadurch gekennzeichnet, dass** aus 2, 3 oder 4 Beobachtungsrichtungen, bevorzugt 4 Beobachtungsrichtungen, beobachtet wird.

9. Verfahren nach Anspruch 5, 6 oder 8 **dadurch gekennzeichnet, dass** die Partikeln von Proben, die in Form von Dispersionen vorliegen unter 4 Winkeln in einer oktagonalen Durchflusszelle beobachtet werden.

10. Verfahren nach Anspruch 9, **dadurch gekennzeichnet, dass** bei zwei Beobachtungsrichtungen der Winkel der Beobachtungsrichtungen zueinander 90° beträgt.

11. Verfahren nach Anspruch 9 oder 10, **dadurch gekennzeichnet, dass** bei 3 oder 4 Beobachtungsrichtungen die Beobachtungsrichtungen in einer Ebene senkrecht zur Förderrichtung der Partikeln liegen.

12. Verfahren nach einem der Ansprüche 1 bis 11, **dadurch gekennzeichnet, dass** die Beobachtung mittels Extinktionsdurchlicht, diffusem Durchlicht, koaxialem Auflicht oder konzentrischem Auflicht, bevorzugt mittels Extinktionsdurchlicht, erfolgt.

13. Verfahren nach einem der Ansprüche 1 bis 12, **dadurch gekennzeichnet, dass** die Kameras in Schritt b) CCD oder CMOS Kameras sind.

14. Verfahren nach einem der Ansprüche 1 bis 13, **dadurch gekennzeichnet, dass** die in Schritt b) erhaltenen Bilder vorverarbeitet werden.

**15.** Verfahren nach einem der Ansprüche 1 bis 14, **dadurch gekennzeichnet, dass** eine Rekonstruktion des Volumens der individuellen Partikeln erfolgt.

**16.** Vorrichtung zur automatisierten Bestimmung der individuellen dreidimensionalen Form von rieselfähigen Partikeln von Proben in Pulverform umfassend:

a) Mittel zum vereinzelnden Dosieren der Partikeln, Mittel zum Ausrichten der Partikeln in Längsachse und Mittel zum automatisierten Fördern der Partikeln entlang einer vorgegebenen Linie;
b) mindestens zwei Kameras zur Beobachtung der ausgerichteten Partikeln; aus mindestens zwei Beobachtungsrichtungen
c) Mittel zur Auswertung der Bilder,

**dadurch gekennzeichnet, dass** die Mittel zum Ausrichten der Partikeln in Längsachse dazu eingerichtet sind, dass die Ausrichtung von Partikeln von Proben in Pulverform gegen zwei plane Anlageflächen, die eine Förderrinne bilden, mit Hilfe der Schwerkraft oder von Zentrifugalkräften, erfolgt.

**17.** Verwendung einer Vorrichtung gemäß Anspruch 16 zur automatisierten Bestimmung der individuellen dreidimensionalen Form von Partikeln von Proben in Pulverform.

**18.** Vorrichtung zur automatisierten Bestimmung der individuellen dreidimensionalen Form von Partikeln von Proben in Form von Dispersionen umfassend:

a) Mittel zum vereinzelnden Dosieren der Partikeln, Mittel zum Ausrichten der Partikeln in Längsachse und Mittel zum automatisierten Fördern der Partikeln entlang einer vorgegebenen Linie;
b) mindestens zwei Kameras zur Beobachtung der ausgerichteten Partikeln; aus mindestens zwei Beobachtungsrichtungen
c) Mittel zur Auswertung der Bilder,

**dadurch gekennzeichnet, dass** die Mittel zum Ausrichten der Partikeln in Längsachse dazu eingerichtet sind, dass die Ausrichtung und automatisierte Förderung von Partikeln von Proben in Form von Dispersionen in einer Strömungszelle zur zweiachsigen Ausrichtung von Partikeln, umfassend eine Zulaufzone für die Probe enthaltend auszurichtende Partikel und einen Auslass für die Probe enthaltend in zwei Achsen ausgerichtete Partikel, wobei ein Fluidelement der Probe mit den Maßen a, b, c in einer Dehnungszone zu einem Fluidelement mit den Maßen a $\times$ n, b/(n $\times$ m), c $\times$ m umgeformt wird, wobei a die Breite, b die Höhe und c die Länge des Fluidelements bedeuten, und n und m von der Geometrie der Strömungszelle abhängige Konstanten, die einem Dehnungsgrad entsprechen, sind, die positive Zahlen > 1 bedeuten, erfolgt.

**19.** Verwendung einer Vorrichtung gemäß Anspruch 18 zur automatisierten Bestimmung der individuellen dreidimensionalen Form von Partikeln von Proben in Form von Dispersionen.

## Claims

**1.** A method for automated determination of the individual three-dimensional shape of flowable particles of samples in powder form, wherein the following steps are carried out in succession:

a) individualized dosing of the particles, alignment of the particles in the longitudinal axis and automated delivery of the particles along a line;
b) observation of the aligned particles from at least two observation directions and image acquisition; wherein the observation is made with the aid of at least two cameras;
c) evaluation of the images,

wherein the alignment of particles of samples in powder form is carried out against two plane surfaces which form a delivery channel, with the aid of gravity or of centrifugal forces, and alignment of the particles is carried out in all axes.

**2.** The method according to claim 1, wherein the individualizing dosing of particles of samples in powder form is carried out with the aid of dosing channels, preferably with the aid of two or more dosing channels connected in succession.

**3.** The method according to claim 1, wherein precision cuvettes or V-channels are used as the delivery channel.

**4.** The method according to one of claims 1 to 3, wherein the automatic delivery of particles of samples in powder form is carried out either by the particles sliding along a line along an intersection line of two surfaces forming a delivery channel, which constitutes slip operation, or by the particles being deposited on a moved intersection line of two surfaces forming a delivery channel, which constitutes entrained delivery.

**5.** A method for automated determination of the individual three-dimensional shape of particles of samples in the form of dispersions, wherein the following steps are carried out in succession:

a) individualized dosing of the particles, alignment of the particles in the longitudinal axis and automated delivery of the particles along a line;
b) observation of the aligned particles from at least two observation directions and image acquisition; wherein the observation is made with the aid of at least two cameras;
c) evaluation of the images,

wherein the alignment and automated delivery of particles of samples in the form of dispersions is carried out in a flow cell for biaxial alignment of particles, comprising a feed zone for the sample comprising particles to be aligned and an outlet for the sample comprising particles aligned in two axes, wherein a fluid element of the sample with the dimensions a, b, c is converted in a stretching zone into a fluid element with the dimensions $a \times n$, $b/(n \times m)$, $c \times m$, where a denotes the width, b denotes the height and c denotes the length of the fluid element, and n and m are constants that correspond to a degree of stretching and depend on the geometry of the flow cell, which denote positive numbers > 1.

**6.** The method according to claim 5, wherein the particles of samples which are present in the form of dispersions are guided through a small thread of flow which is embedded in a flow through a larger opening, wherein the flow through the larger opening constitutes an envelope stream.

**7.** The method according to claim 5 or 6, wherein the automatic delivery of the particles of samples which are present in the form of dispersions is carried out by means of a pump or a pressure gradient.

**8.** The method according to one of claims 1 to 7, wherein observation is carried out from 2, 3 or 4 observation directions, preferably 4 observation directions.

**9.** The method according to claim 5, 6 or 8, wherein the particles of samples which are present in the form of dispersions are observed at 4 angles in an octagonal flow cell.

**10.** The method according to claim 9, wherein the angle between the observation directions is 90° when there are two observation directions.

**11.** The method according to claim 9 or 10, wherein the observation directions lie in a plane perpendicular to the delivery direction of the particles when there are 3 or 4 observation directions.

**12.** The method according to one of claims 1 to 11, wherein the observation is carried out by means of extinction transmitted light, diffuse transmitted light, coaxial direct light or concentric direct light, preferably by means of extinction transmitted light.

**13.** The method according to one of claims 1 to 12, wherein the cameras in step b) are CCD or CMOS cameras.

**14.** The method according to one of claims 1 to 13, wherein the images obtained in step b) are preprocessed.

**15.** The method according to one of claims 1 to 14, wherein a reconstruction of the volume of the individual particles is carried out.

**16.** An apparatus for automated determination of the individual three-dimensional shape of flowable particles of samples in powder form, comprising:

a) means for individualized dosing of the particles, means for alignment of the particles in the longitudinal axis and means for automated delivery of the particles along a predetermined line;
b) at least two cameras for observation of the aligned particles; from at least two observation directions
c) means for evaluation of the images,

wherein the means for alignment of the particles in the longitudinal axis are set up such that the alignment of particles of samples in powder form is carried out against two plane surfaces which form a delivery channel, with the aid of gravity or with centrifugal forces.

17. The use of the apparatus according to claim 16 for automated determination of the individual three-dimensional shape of particles of samples in powder form.

18. An apparatus for automated determination of the individual three-dimensional shape of particles of samples in the form of dispersions, comprising:

a) means for individualized dosing of the particles, means for alignment of the particles in the longitudinal axis and means for automated delivery of the particles along a predetermined line;
b) at least two cameras for observation of the aligned particles; from at least two observation directions
c) means for evaluation of the images,

wherein the means for alignment of the particles in the longitudinal axis are set up such that the alignment and automated delivery of particles of samples in the form of dispersions is carried out in a flow cell for biaxial alignment of particles, comprising a feed zone for the sample comprising particles to be aligned and an outlet for the sample comprising particles aligned in two axes, wherein a fluid element of the sample with the dimensions a, b, c is converted in a stretching zone into a fluid element with the dimensions $a \times n$, $b/(n \times m)$, $c \times m$, where a denotes the width, b denotes the height and c denotes the length of the fluid element, and n and m are constants that correspond to a degree of stretching and depend on the geometry of the flow cell, which denote positive numbers > 1.

19. The use of the apparatus according to claim 18 for automated determination of the individual three-dimensional shape of particles of samples in the form of dispersions.

**Revendications**

1. Procédé de détermination automatisée de la forme tridimensionnelle de particules individuelles d'échantillons présentant la forme d'une poudre fluide, dans lequel les étapes suivantes sont réalisées successivement :

a) dosage une à une des particules, alignement des particules suivant leur axe longitudinal et transport automatisé des particules suivant une ligne,
b) observation des particules alignées suivant au moins deux directions d'observation, l'observation ayant lieu à l'aide d'au moins deux caméras, et
c) évaluation des images,

**caractérisé en ce que**
l'alignement des particules d'échantillon présentant la forme d'une poudre par rapport à deux surfaces de pose planes qui forment une rigole de transport s'effectue à l'aide de la gravité ou de forces centrifuges et **en ce que** l'alignement des particules s'effectue suivant tous les axes.

2. Procédé selon la revendication 1, **caractérisé en ce que** le dosage de particules individuelles d'échantillons présentant la forme d'une poudre s'effectue à l'aide de rigoles de dosage et de préférence à l'aide de deux ou plusieurs rigoles de dosage raccordées à la suite les unes des autres.

3. Procédé selon la revendication 1, **caractérisé en ce que** les rigoles de transport utilisées sont des cuvettes ou rigoles en V de précision.

4. Procédé selon l'une des revendications 1 à 3, **caractérisé en ce que** le transport automatisé des particules d'échantillons présentant la forme d'une poudre s'effectue soit par glissement des particules le long d'une ligne, à savoir une ligne de coupe de deux surfaces formant une rigole de transport, ce qui représente un déplacement par patinage, soit par dépôt des particules sur une ligne de coupe en déplacement de deux surfaces formant une rigole de transport, ce qui représente un transport par entraînement.

5. Procédé de détermination automatisée de la forme tridimensionnelle de particules individuelles d'échantillons présentant la forme d'une dispersion, dans lequel les étapes suivantes sont réalisées successivement :

   a) dosage une à une des particules, alignement des particules suivant leur axe longitudinal et transport automatisé des particules suivant une ligne,
   b) observation des particules alignées suivant au moins deux directions d'observation et saisie d'images, l'observation ayant lieu à l'aide d'au moins deux caméras, et
   c) évaluation des images,

   **caractérisé en ce que**
   l'alignement et le transport automatisé des particules d'échantillon présentant la forme d'une dispersion s'effectue dans une cellule d'écoulement servant à aligner les particules suivant deux axes et comprenant une zone d'entrée de l'échantillon contenant les particules à aligner et une sortie de l'échantillon contenant des particules alignées suivant deux axes,
   **en ce qu'**un élément fluide de l'échantillon de dimensions a, b, c est déformé dans une zone d'allongement en un élément fluide de dimensions axn, b/(nxm), cxm, a représentant la largeur, b la hauteur et c la longueur de l'élément fluide, n et m étant des constantes dépendant de la géométrie de la cellule d'écoulement, correspondant à un degré d'allongement et étant des nombres positifs > 1.

6. Procédé selon la revendication 5, **caractérisé en ce que** les particules d'échantillons qui présentent la forme d'une dispersion sont guidées par un petit filet d'écoulement incorporé dans un écoulement par une ouverture plus grande, l'écoulement par l'ouverture plus grande représentant un écoulement d'enveloppe.

7. Procédé selon les revendications 5 ou 6, **caractérisé en ce que** le transport automatisé des particules d'échantillons qui présentent la forme d'une dispersion s'effectue au moyen d'une pompe ou d'un gradient hydraulique.

8. Procédé selon l'une des revendications 1 à 7, **caractérisé en ce que** l'observation s'effectue suivant 2, 3 ou 4 directions d'observation et de préférence suivant 4 directions d'observation.

9. Procédé selon les revendications 5, 6 ou 8, **caractérisé en ce que** les particules d'échantillons qui présentent la forme d'une dispersion sont observées suivant 4 angles dans une cellule d'écoulement octogonale.

10. Procédé selon la revendication 9, **caractérisé en ce que** dans le cas de deux directions d'observation, l'angle entre les directions d'observation est de 90°.

11. Procédé selon les revendications 9 ou 10, **caractérisé en ce que** dans le cas de 3 ou 4 directions d'observation, les directions d'observation sont situées dans un plan perpendiculaire à la direction de transport des particules.

12. Procédé selon l'une des revendications 1 à 11, **caractérisé en ce que** l'observation s'effectue par extinction de lumière transmise, diffusion de lumière transmise, éclairage coaxial ou concentrique et de préférence par extinction de lumière transmise.

13. Procédé selon l'une des revendications 1 à 12, **caractérisé en ce que** les caméras de l'étape b) sont des caméras CCD ou CMOS.

14. Procédé selon l'une des revendications 1 à 13, **caractérisé en ce que** les images obtenues à l'étape b) subissent un prétraitement.

15. Procédé selon l'une des revendications 1 à 14, **caractérisé en ce que** le volume des particules individuelles est reconstruit.

16. Ensemble de détermination automatisée de la forme tridimensionnelle de particules individuelles d'échantillons présentant la forme d'une poudre fluide, comprenant :

   a) des moyens de dosage une à une des particules, des moyens d'alignement des particules suivant leur axe longitudinal et des moyens de transport automatisé des particules suivant une ligne prédéterminée,

b) au moins deux caméras d'observation des particules, alignées suivant au moins deux directions d'observation, et

c) des moyens d'évaluation des images,

**caractérisé en ce que**

les moyens d'alignement des particules suivant l'axe longitudinal sont configurés de telle sorte que l'alignement des particules d'échantillons présentant la forme d'une poudre par rapport à deux surfaces de pose planes qui forment une rigole de transport s'effectue à l'aide de la gravité ou de forces centrifuges.

17. Utilisation d'un ensemble selon la revendication 16 pour la détermination automatisée de la forme tridimensionnelle de particules individuelles d'échantillons présentant la forme d'une poudre.

18. Procédé de détermination automatisée de la forme tridimensionnelle de particules individuelles d'échantillons présentant la forme d'une dispersion, comprenant :

a) des moyens de dosage une à une des particules, des moyens d'alignement des particules suivant leur axe longitudinal et des moyens de transport automatisé des particules suivant une ligne prédéterminée,
b) au moins deux caméras d'observation des particules alignées suivant au moins deux directions d'observation et
c) des moyens d'évaluation des images,

**caractérisé en ce que**

les moyens d'alignement des particules d'échantillon suivant leur axe longitudinal sont configurés de telle sorte que l'alignement et le transport automatisé des particules d'échantillons présentant la forme d'une dispersion s'effectuent dans une cellule d'écoulement servant à aligner les particules suivant deux axes, comprenant une zone d'entrée de l'échantillon contenant les particules à aligner et une sortie de l'échantillon contenant des particules alignées suivant deux axes,

**en ce qu'**un élément fluide de l'échantillon de dimensions a, b, c est déformé dans une zone d'allongement en un élément fluide de dimensions $axn$, $b/(nxm)$, $cxm$, a représentant la largeur, b la hauteur et c la longueur de l'élément fluide, n et m étant des constantes dépendant de la géométrie de la cellule d'écoulement, correspondant à un degré d'allongement et étant des nombres positifs > 1.

19. Utilisation d'un ensemble selon la revendication 18 pour la détermination automatisée de la forme tridimensionnelle de particules d'échantillons présentant la forme d'une dispersion.

# Fig. 1

1.1    1.2    1,3

2

3

Fig. 2A

Fig. 2B

Fig. 2C

# Fig. 3

EP 1 955 045 B1

**Fig. 4**

# Fig. 5 (Var. 1)

Fig. 6 (Var. 1`)

# Fig. 7 (Var. 1b)

# Fig. 8 (Var. 2)

A = 135°  
Q = 45°  
H = 0°  
V = -90°

## Fig. 9 (Var. 2b)

A = 135°

2

Q = 45°

H = 0°

V = -90°

# Fig. 10 (Var. 2c)

A = 135°

2a

Q = 45°

H = 0°

V = -90°

2b

## Fig. 11 (Var. 3-2)

Fig. 12 (Var. 3-2b)

Fig. 13 (Var. 3-3)

# Fig. 14 (Var. 3-4)

# Fig. 15 (Var. 3-4b)

# Fig. 16 (Var. 4)

**Fig. 17 (Var. 5)**

Fig. 18 (Var. 5b)

Fig. 19

EP 1 955 045 B1

EP 1 955 045 B1

## Fig. 20

EP 1 955 045 B1

# Fig. 21

51

# Fig. 22

Fig. 23 (Var. 4b)

**Fig. 24**

1

2

3

4

5

6

7

8

9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2445148 A
- WO 0211065 A2
- EP 1464949 A2
- US 5644388 A
- US 6049381 A
- US 4999513 A
- EP 1662247 A1
- WO 2005062022 A
- US 4975863 A
- US 20030053065 A1
- EP 2004014603 W

**In der Beschreibung aufgeführte Nicht-Patentliteratur**

- Handbuch der Mechanischen Verfahrenstechnik
- **KACHEL V et al.** *Journal of Histochemistry and Cytochemistry,* 1977, vol. 25 (7), 774-780
- **R. WEICHERT ; D. HULLER.** Volumenbestimmung und Formerkennung unregelmäßig geformter Partikeln mittels dreidimensionaler Bildanalyse. *Nürnberg, 2. Europ. Symposium Partikelmesstechnik,* 1979, 266-272
- **KACHEL et al.** UNIFORM LATERAL ORIENTATION, CAUSED BY FLOW FORCES, OF FLAT PARTICLES IN FLOW-THROUGH SYSTEMS. *THE JOURNAL OF HISTOCHEMISTRY AND CYTOCHEMISTRY,* 1977, vol. 25 (7), 774-780
- 1D und 2D Abbildende Prozesssonden: Theorie und Praxis. *Vortrag von Dr. M. Schäfer auf dem Fachausschuss Partikelmesstechnik am 23.02.2005 in Würzburg,* 23. Februar 2005
- **WEICHERT, R. ; HULLER, D.** Volumenbestimmung und Formerkennung unregelmäßig geformter Partikeln mittels dreidimensionaler Bildanalyse. *Nürnberg, 2. Europ. Symposium Partikelmesstechnik,* 1979, 266-272
- **MICHAEL SCHÄFER.** Digital Optics: Some Remarks on the Accuracy of Particle Image Analysis. *Part.Part.Syst.Charact.,* Juli 2002, vol. 19 (3), 158-168